(19) Europäisches Patentamt
European Patent Office
Office européen des brevets

(11) Veröffentlichungsnummer: **0 556 789 A2**

# (12) EUROPÄISCHE PATENTANMELDUNG

(21) Anmeldenummer: **93102456.6**

(22) Anmeldetag: **17.02.93**

(51) Int. Cl.5: **C07D 235/08**, C07D 235/18, C07D 403/10, C07D 403/04, C07D 403/14, C07D 471/04, A61K 31/415, //(C07D471/04, 235:00,221:00)

(30) Priorität: **19.02.92 DE 4204968**
**15.06.92 DE 4219534**

(43) Veröffentlichungstag der Anmeldung:
**25.08.93 Patentblatt 93/34**

(84) Benannte Vertragsstaaten:
**AT BE CH DE DK ES FR GB GR IE IT LI LU NL PT SE**

(71) Anmelder: **Dr. Karl Thomae GmbH**

**D-88397 Biberach(DE)**

(72) Erfinder: **Hauel, Norbert, Dr. Dipl.-Chem.**
**Marderweg 12**
**W-7957 Schemmerhofen(DE)**

Erfinder: **Narr, Berthold, Dr. Dipl.-Ing.**
**Obere Au 5**
**W-7950 Biberach 1(DE)**
Erfinder: **Ries, Uwe, Dr. Dipl.-Chem.**
**Dunantstrasse 10**
**W-7950 Biberach 1(DE)**
Erfinder: **van Meel, Jacques, Dr. Pharm.**
**Schubertweg 4**
**W-7951 Mittelbiberach(DE)**
Erfinder: **Wienen, Wolfgang, Dr. Dipl.-Biol.**
**Am Schiessberg 28**
**W-7951 Äpfingen(DE)**
Erfinder: **Entzeroth, Michael, Dr. Dipl.-Chem.**
**Sebastian-Sailer-Strasse 44**
**W-7951 Warthausen(DE)**

## (54) Substituierte Biphenylylderivate, diese Verbindungen enthaltende Arzneimittel und Verfahren zu ihrer Herstellung.

(57) Die Erfindung betrifft substituierte Biphenylylderivate der allgemeinen Formel

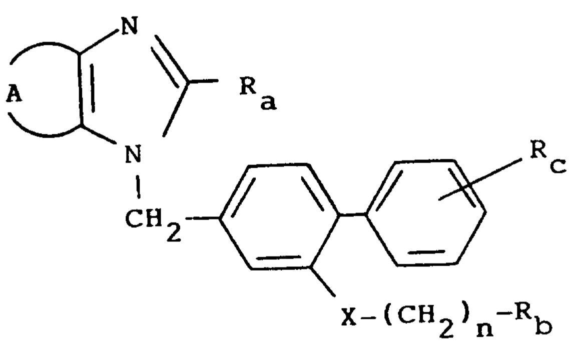

,(I)

in der
$R_a$ bis $R_c$, X und n wie im Anspruch 1 definiert sind, deren Gemische von Stellungsisomerenmeren und deren Salze, welche wertvolle Eigenschaften aufweisen.

EP 0 556 789 A2

Die vorliegende Erfindung betrifft neue substituierte Biphenylylderivate der allgemeinen Formel

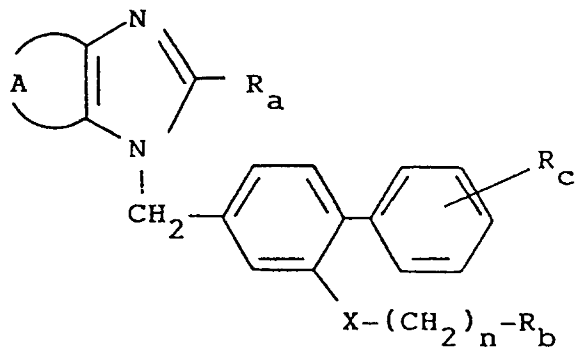

, (I)

deren Gemische von Stellungsisomeren sowie deren Salze, insbesondere für die pharmazeutische Anwendung deren physiologisch verträgliche Salze mit anorganischen oder organischen Säuren oder Basen, welche wertvolle pharmakologische Eigenschaften aufweisen, insbesondere Angiotensin-antagonistische Wirkungen, vorzugsweise Angiotensin-II-antagonistische Wirkungen.

In der obigen allgemeinen Formel bedeuten

n die Zahl 0, 1, 2 oder 3 und

X eine Bindung oder

n die Zahl 1, 2 oder 3 und

X eine gegebenenfalls durch eine Alkylgruppe mit 1 bis 3 Kohlenstoffatomen substituierte Iminogruppe, ein Sauerstoff- oder Schwefelatom,

A eine 1,4-Butadienylengruppe, die durch die Reste $R_1$ und $R_2$ substituiert ist und in der zusätzlich eine unsubstituierte Methingruppe durch ein Stickstoffatom ersetzt sein kann, wobei

$R_1$ ein Wasserstoff-, Fluor-, Chlor- oder Bromatom, eine Trifluormethylgruppe oder eine Alkylgruppe mit 1 bis 3 Kohlenstoffatomen und

$R_2$ ein Wasserstoffatom,

eine Alkylgruppe mit 1 bis 3 Kohlenstoffatomen,

eine Alkoxygruppe mit 2 bis 5 Kohlenstoffatomen, die in 2-, 3-, 4- oder 5-Position durch eine Imidazolyl-, Benzimidazolyl- oder Tetrahydrobenzimidazolylgruppe substituiert ist,

eine Alkanoylaminogruppe mit 2 bis 5 Kohlenstoffatomen im Alkanoylteil oder eine Benzolsulfonylaminogruppe, welche beide am Stickstoffatom durch eine Alkylgruppe mit 1 bis 3 Kohlenstoffatomen substituiert sein können,

eine Phthalimino- oder Homophthaliminogruppe, wobei eine Carbonylgruppe in einer Phthaliminogruppe durch eine Methylengruppe ersetzt sein kann,

eine 5-, 6- oder 7-gliedrige Alkyleniminogruppe, in welcher eine Methylengruppe durch eine Carbonyl- oder Sulfonylgruppe ersetzt sein kann,

eine Glutarsäureiminogruppe, in der die n-Propylengruppe durch ein oder zwei Alkylgruppen mit jeweils 1 bis 3 Kohlenstoffatomen oder durch eine Tetramethylen- oder Pentamethylengruppe substituiert sein kann,

eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,

eine gegebenenfalls in 1-Stellung durch eine Alkylgruppe mit 1 bis 6 Kohlenstoffatomen oder eine Cycloalkylgruppe mit 3 bis 7 Kohlenstoffatomen substituierte Benzimidazol-2-yl-gruppe, wobei der Phenylkern in einer vorstehend erwähnten Benzimidazol-2-ylgruppe zusätzlich durch ein Fluoratom, eine Methyl- oder Trifluormethylgruppe substituiert sein kann, eine Imidazo[2,1-b]thiazol-6-yl-, Imidazo[1,2-a]pyridin-2-yl-, 5,6,7,8-Tetrahydro-imidazo[1,2-a]pyridin-2-yl-, Imidazo[1,2-a]pyrimidin-2-yl-, Imidazo[4,5-b]pyridin-2-yl-, Imidazo[4,5-c]pyridin-2-yl-, Imidazo[1,2-c]pyrimidin-2-yl-, Imidazo[1,2-a]pyrazin-2-yl-, Imidazo[1,2-b]-pyridazin-2-yl-, Imidazo[4,5-c]pyridin-2-yl-, Purin-8-yl-, Imidazo[4,5-b]pyrazin-2-yl-, Imidazo[4,5-c]pyridazin-2-yl- oder Imidazo[4,5-d]pyridazin-2-yl-gruppe,

einen über ein Kohlenstoffatom gebundenen Pyrrolidin-, Piperidin- oder Pyridinring, wobei an den Pyridinring über zwei benachbarte Kohlenstoffatome ein Phenylrest ankondensiert und eine zum N-Atom benachbarte Methylengruppe in einem Pyrrolidin- oder Piperidinring durch eine Carbonyl- oder Sulfonylgruppe ersetzt sein kann,

eine gegebenenfalls in 2-Stellung durch eine Alkylgruppe mit 1 bis 6 Kohlenstoffatomen oder durch eine

Phenylgruppe substituierte Imidazol-4-yl-Gruppe, die in 1-Stellung durch eine Alkylgruppe mit 1 bis 7 Kohlenstoffatomen, die in 2-, 3-, 4-, 5-, 6- oder 7-Stellung durch eine Carboxy-, Alkoxycarbonyl-, Aminocarbonyl-, Alkylaminocarbonyl-, Dialkylaminocarbonyl-, Morpholinocarbonyl-, Thiomorpholinocarbonyl- oder 1-Oxidothiomorpholinocarbonylgruppe substituiert sein kann, durch eine Alkylgruppe mit 1 bis 4 Kohlenstoffatomen, die in 2-, 3- oder 4-Stellung durch eine Hydroxy-, Alkoxy- oder Imidazol-1-yl-Gruppe substituiert ist, durch eine Alkylgruppe, die durch eine Trifluormethylgruppe, durch eine Cycloalkylgruppe mit 3 bis 7 Kohlenstoffatomen oder durch eine gegebenenfalls durch Fluor- oder Chloratome, durch Trifluormethyl-, Methyl- oder Methoxygruppen mono- oder disubstituierte Phenylgruppe substituiert ist, durch eine durch zwei Phenylgruppen substituierte Alkylgruppe oder durch eine Cycloalkylgruppe mit 3 bis 7 Kohlenstoffatomen substituiert ist, wobei, soweit nichts anderes erwähnt wurde, die vorstehend erwähnten Alkyl- und Alkoxyteile jeweils 1 bis 3 Kohlenstoffatome enthalten können,
eine in vivo in eine Carboxygruppe überführbare Gruppe,
eine Carbonylgruppe, die durch eine Hydroxygruppe, durch eine Alkoxygruppe mit 1 bis 6 Kohlenstoffatomen, wobei die Methoxygruppe zusätzlich durch eine Alkanoyloxy-, Alkoxycarbonyloxy- oder Cycloalkoxycarbonyloxygruppe substituiert ist, oder durch eine gegebenenfalls durch Alkylgruppen mono- oder disubsituierte Aminogruppe substituiert ist, wobei in den vorstehend erwähnten Gruppen der Alkanoylteil 2 oder 3 Kohlenstoffatome, der Alkyl- und Alkoxyteil jeweils 1 bis 6 Kohlenstoffatome und der Cycloalkoxyteil 5 bis 7 Kohlenstoffatome enthalten kann, oder
eine $R_5$-$NR_4$-CO-$NR_3$-Gruppe, in der

$R_3$ ein Wasserstoffatom, eine Alkylgruppe mit 1 bis 5 Kohlenstoffatomen, eine Cyclohexyl- oder Benzylgruppe,

$R_4$ ein Wasserstoffatom, eine Alkylgruppe mit 1 bis 6 Kohlenstoffatomen, eine Allyl-, Cyclohexyl-, Benzyl- oder Phenylgruppe,

$R_5$ ein Wasserstoffatom oder eine Alkylgruppe mit 1 bis 3 Kohlenstoffatomen oder

$R_4$ und $R_5$ zusammen mit dem dazwischen liegenden Stickstoffatom eine unverzweigte Cycloalkyleniminogruppe mit 4 bis 6 Kohlenstoffatomen oder eine Morpholinogruppe oder

$R_3$ und $R_4$ zusammen eine Alkylengruppe mit 2 oder 3 Kohlenstoffatomen darstellen,

$R_a$ eine Alkylgruppe mit 1 bis 5 Kohlenstoffatomen, eine Cycloalkylgruppe mit 3 bis 5 Kohlenstoffatomen, eine Alkoxy-, Alkylthio- oder Alkylaminogruppe mit jeweils 1 bis 3 Kohlenstoffatomen in jedem Alkylteil,

$R_b$ eine in vivo in eine Carboxygruppe überführbare Gruppe, eine Carboxy-, Cyano-, Hydroxysulfonyl-, 1H-Tetrazolyl-, 1-Triphenylmethyl-tetrazolyl- oder 2-Triphenylmethyl-tetrazolylgruppe, eine Alkancarbonylaminosulfonyl-, Benzoylaminosulfonyl-, Alkansulfonylaminocarbonyl-, Trifluormethansulfonylaminocarbonyl- oder Phenylsulfonylaminocarbonylgruppe oder auch, wenn n die Zahl 1 und X eine Bindung darstellen, eine Bis(hydroxycarbonyl)methyl- oder Bis(alkoxcarbonyl)methylgruppe, wobei in den vorstehend erwähnten Gruppen die Alkyl- und Alkoxyteile jeweils 1 bis 4 Kohlenstoffatome enthalten können, und

$R_c$ ein Wasserstoff-, Fluor-, Chlor- oder Bromatom, eine Alkylgruppe mit 1 bis 4 Kohlenstoffatomen, eine Alkoxy-, Nitro-, Amino-, Alkylamino- oder Dialkylaminogruppe mit jeweils 1 bis 3 Kohlenstoffatomen in jedem Alkylteil,

wobei der Phenylkern der vorstehend erwähnten Phenylgruppen jeweils durch Chlor- oder Bromatome, Methyl- oder Methoxygruppen mono- oder disubstituiert sein kann und die Substituenten gleich oder verschieden sein können.

Unter dem vorstehend erwähnten Begriff "eine in vivo in eine Carboxygruppe überführbare Gruppe" sind beispielsweise deren Ester der Formeln

- CO - OR',
- CO - O - (HCR'') - O - CO - R''' und
- CO - O - (HCR'') - O - CO - OR'''

in denen
R' eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,
R'' ein Wasserstoffatom oder eine Methylgruppe und
R''' eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe bedeuten, zu verstehen.

Die neuen Verbindungen der obigen Formel I weisen wertvolle Eigenschaften auf. So weisen die Verbindungen der Formel I, in der $R_b$ eine in vivo in eine Carboxygruppe überführbare Gruppe, eine Carboxy-, Hydroxysulfonyl-, 1H-Tetrazolyl-, Alkancarbonylaminosulfonyl-, Benzoylaminosulfonyl-, Alkansulfonylaminocarbonyl-, Trifluormethansulfonylaminocarbonyl- oder Phenylsulfonylaminocarbonylgruppe oder auch, wenn n die Zahl 1 und X eine Bindung darstellen, eine Bis(hydroxycarbonyl)methyl- oder Bis(alkoxcarbonyl)methylgruppe bedeutet, insbesondere wertvolle pharmakologische Eigenschaften auf, da diese Angiotensin-Antagonisten, insbesondere Angiotensin-II-Antagonisten, darstellen. Die übrigen Verbindungen der allgemeinen Formel I stellen wertvolle Zwischenprodukte zur Herstellung der vorstehend erwähnten Verbindungen dar.

Gegenstand der vorliegenden Erfindung sind somit die neuen Benzimidazol-1-yl-, Imidazo[4,5-b]pyridin-1-yl-, Imidazo[4,5-c]pyridin-1-yl-, Imidazo[4,5-b]pyridin-3-yl- und Imidazo[4,5-c]pyridin-3-yl-biphenylmethyl-Derivate der obigen allgemeinen Formel I, deren Gemische von Stellungsisomeren und deren Salze, insbesondere für die pharmazeutische Anwendung, deren verträglichen Salze, und Verfahren zu ihrer Herstellung.

Ein weiterer Gegenstand der vorliegenden Erfindung sind somit neue Arzneimittel, welche eine der oben erwähnten pharmakologisch wirksamen Verbindungen der allgemeinen Formel I oder ein entsprechendes physiologisch verträgliches Salz enthalten und insbesondere zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen geeignet sind.

Für die bei der Definition der Reste $R_a$ bis $R_b$ und X eingangs erwähnten Bedeutungen kommt beispielsweise

Für $R_a$ die Bedeutung der Methyl-, Ethyl-, n-Propyl-, Isopropyl-, n-Butyl-, 1-Methyl-n-propyl-, 2-Methyl-n-propyl-, tert.Butyl-, n-Pentyl-, 1-Methyl-1-butyl-, 2-Methyl-1-butyl-, 3-Methyl-1-butyl-, 1,1-Dimethyl-1-propyl-, 2,2-Dimethyl-1-propyl-, Cyclopropyl-, Cyclobutyl-, Cyclopentyl-, Methoxy-, Ethoxy-, n-Propoxy-, Isopropoxy-, Methylthio-, Ethylthio-, n-Propylthio-, Isopropylthio-, Methylamino-, Ethylamino-, n-Propylamino- oder Isopropylaminogruppe,

für $R_b$ die Hydroxycarbonyl-, Hydroxysulfonyl-, Cyano-, 1H-Tetrazolyl-, 1-Triphenylmethyl-tetrazolyl-, 2-Triphenylmethyl-tetrazolyl-, Trifluormethansulfonylaminocarbonyl-, Methansulfonylaminocarbonyl-, Ethansulfonylaminocarbonyl-, n-Propansulfonylaminocarbonyl-, Isopropansulfonylaminocarbonyl-, Phenylsulfonylaminocarbonyl-, 4-Fluorphenylsulfonylaminocarbonyl-, 4-Chlorphenylsulfonylaminocarbonyl-, 4-Bromphenylsulfonylaminocarbonyl-, 4-Methylphenylsulfonylaminocarbonyl-, 4-Methoxyphenylsulfonylaminocarbonyl-, Methylcarbonylaminosulfonyl-, Ethylcarbonylaminosulfonyl-, n-Propylcarbonylaminosulfonyl-, n-Butylcarbonylaminosulfonyl-, Benzoylaminosulfonyl-, Bis(hydroxycarbonyl)-methyl-, Bis(methoxycarbonyl)methyl-, Bis(ethoxycarbonyl)-methyl-, Bis(n-propoxycarbonyl)methyl- oder Bis(isopropoxycarbonyl)methyl-gruppe,

für $R_c$ die des Wasserstoff-, Fluor-, Chlor- oder Bromatoms, der Methyl-, Ethyl-, n-Propyl-, Isopropyl-, Isobutyl-, n-Butyl-, 1-Methyl-n-propyl-, 2-Methyl-n-propyl-, tert.Butyl-, Methoxy-, Ethoxy-, n-Propoxy-, Isopropoxy-, Nitro-, Amino-, Methylamino-, Ethylamino-, n-Propylamino-, Isopropylamino-, Dimethylamino-, Diethylamino-, Di-n-propylamino-, Diisopropylamino- oder N-Ethyl-methylaminogruppe,

für X die einer Bindung, des Sauerstoff- oder Schwefelatoms, der Imino-, Methylimino-, Ethylimino-, n-Propylimino- oder Isopropyliminogruppe,

für $R_1$ die des Wasserstoff-, Fluor-, Chlor- oder Bromatoms, der Methyl-, Ethyl-, n-Propyl-, Isopropyl- oder Trifluormethylgruppe,

für $R_2$ die des Wasserstoffatoms, der Acetylamino-, Propionylamino-, Butanoylamino-, Pentanoylamino-, Benzoylamino-, N-Acetyl-methylamino-, N-Propionyl-methylamino-, N-Butanoyl-methylamino-, N-Pentanoylmethylamino-, N-Benzoyl-methylamino-, N-Acetyl-ethylamino-, N-Propionyl-ethylamino-, N-Butanoyl-ethylamino-,N-Pentanoyl-ethylamino-, N-Benzoyl-ethylamino-, N-Acetyl-isopropylamino-, N-Propionyl-n-propylamino-, N-Butanoyl-n-propylamino-, N-Pentanoyl-isopropylamino-, N-Benzoyl-isopropylamino-, 2-(Imidazol-1-yl)-ethoxy-, 3-(Imidazol-1-yl)-propoxy-, 4-(Imidazol-1-yl)-butoxy-, 5-(Imidazol-1-yl)-pentoxy-, 2-(Benzimidazol-1-yl)-ethoxy-, 3-(Benzimidazol-1-yl)-propoxy-, 4-(Benzimidazol-1-yl)-butoxy-, 5-(Benzimidazol-1-yl)-pentoxy-, 2-(Tetrahydrobenzimidazol-1-yl)-ethoxy-, 3-(Tetrahydrobenzimidazol-1-yl)-propoxy-, 4-(Tetrahydrobenzimidazol-1-yl)-butoxy-, 5-(Tetrahydrobenzimidazol-1-yl)-pentoxy-, Acetylamino-, Propionylamino-, Butanoylamino-, Isobutanoylamino-, Pentanoylamino-, Phthalimino-, Homophthalimino-, 1-Oxo-isoindolin-2-yl-, Pyrrolidino-, Piperidino-, Hexamethylenimino-, 2-Oxo-pyrrolidino-, 2-Oxo-piperidino-, 2-Oxo-hexamethylenimino-, Propansultam-1-yl-, Butansultam-1-yl-, Pentansultam-1-yl-, Glutarimino-, 3,3-Tetramethylen-glutarimino-, 3,3-Pentamethylen-glutarimino-,

2,2-Dimethyl-glutarimino-, 3-Methyl-glutarimino-, 3,3-Dimethyl-glutarimino-, 3-Ethyl-glutarimino-, 3-Ethyl-3-methyl-glutarimino-, 1,3-Cyclopentandicarbonylimino-, 2,4-Dimethyl-glutarimino-, 2,4-Di-n-propyl-glutarimino-, Maleinsäureimido-, 2-Methyl-maleinsäureimido-, 2-Phenyl-maleinsäureimido-, 2,3-Dimethyl-maleinsäureimido, 3-Methyl-2-phenyl-maleinsäureimido-, 2,3-Diphenyl-maleinsäureamido-, Pyrrolidin-2-yl-, Pyrrolidin-2-on-5-yl-, Piperidin-2-yl-, Piperidin-2-on-1-yl-,Piperidin-2-on-6-yl, Pyridin-2-yl-, Chinolin-2-yl-, Isochinolin-1-yl-, Isochinolin-3-yl-, 1-Methyl-imidazol-4-yl-, 1-Ethyl-imidazol-4-yl-, 1-n-Propyl-imidazol-4-yl-, 1-Isopropyl-imidazol-4-yl-, 1-n-Butyl-imidazol-4-yl-, 1-Isobutyl-imidazol-4-yl-, 1-n-Pentyl-imidazol-4-yl-, 1-Isoamyl-imidazol-4-yl-, 1-n-Hexyl-imidazol-4-yl-, 1-n-Hexyl-2-methyl-imidazol-4-yl-, 1-(1-Methyl-n-pentyl)-imidazol-4-yl-, 1-(1-Ethyl-n-butyl)-imidazol-4-yl-, 1-(1-Methyl-n-hexyl)-imidazol-4-yl-, 1-(1-Ethyl-n-pentyl)-imidazol-4-yl-, 1-(1-n-Propyl-n-butyl)-imidazol-4-yl-, 1-n-Heptyl-imidazol-4-yl-, 1-Ethyl-2-methyl-imidazol-4-yl-,1-n-Propyl-2-methyl-imidazol-4-yl-, 1-Isopropyl-2-methyl-imidazol-4-yl-, 1-n-Butyl-2-methyl-imidazol-4-yl-, 1-Isobutyl-2-methyl-imidazol-4-yl-, 1-n-Pentyl-2-methyl-imidazol-4-yl-, 1-Isoamyl-2-methyl-imidazol-4-yl-, 1-n-Hexyl-2-methyl-imidazol-4-yl-, 1-n-Heptyl-2-methyl-imidazol-4-yl-, 1-Cyclopropylmethyl-imidazol-4-yl-, 1-Cyclobutylmethyl-imidazol-4-yl-, 1-Cyclopentylmethyl-imidazol-4-yl, 1-Cyclohexylmethyl-imidazol-4-yl-,1-Cycloheptylmethyl-imidazol-4-yl-, 1-(2-Cyclopropylethyl)-imidazol-4-yl-, 1-(2-Cyclobutylethyl)-imidazol-4-yl-, 1-(2-Cyclopentylethyl)-imidazol-4-yl-, 1-(2-Cyclohexylethyl)-imidazol-4-yl-, 1-(2-Cycloheptylethyl)-imidazol-4-yl-, 1-(3-Cyclopropylpropyl)-imidazol-4-yl-, 1-(3-Cyclobutylpropyl)-imidazol-4-yl-, 1-(3-Cyclopentylpropyl)-imidazol-4-yl-, 1-(3-Cyclohexylpropyl)-imidazol-4-yl-, 1-(3-Cycloheptylpropyl)-imidazol-4-yl-, 1-(2,2,2-Trifluorethyl)-imidazol-4-yl-, 1-(3,3,3-Trifluorpropyl)-imidazol-4-yl-, 1-Benzyl-imidazol-4-yl-,1-(2-Phenylethyl)-imidazol-4-yl-, 1-(3-Phenylpropyl)-imidazol-4-yl-, 1-(4-Fluor-benzyl)-imidazol-4-yl-, 1-(4-Chlor-benzyl)-imidazol-4-yl-, 1-(3-Chlor-benzyl)-imidazol-4-yl-, 1-(4-Trifluormethyl-benzyl)-imidazol-4-yl-, 1-(3-Methyl-benzyl)-imidazol-4-y1-, 1-(4-Methyl-benzyl)-imidazol-4-yl-, 1-(3-Methoxy-benzyl)-imidazol-4-yl, 1-(4-Methoxy-benzyl)-imidazol-4-yl-, 1-(3,4-Dimethoxy-benzyl)-imidazol-4-yl-, 1-(3,5-Dimethoxy-benzyl)-imidazol-4-yl-, 1-Cyclopropylmethyl-2-methyl-imidazol-4-yl-, 1-Cyclobutylmethyl-2-methyl-imidazol-4-yl-, 1-Cyclopentylmethyl-2-methyl-imidazol-4-yl-, 1-Cyclohexylmethyl-2-methyl-imidazol-4-yl-, 1-Cycloheptylmethyl-2-methyl-imidazol-4-yl-, 1-(2-Cyclopropylethyl)-2-methyl-imidazol-4-yl, 1-(2-Cyclobutylethyl)-2-methyl-imidazol-4-yl-, 1-(2-Cyclopentylethyl)-2-methyl-imidazol-4-yl-, 1-(2-Cyclohexylethyl)-2-methyl-imidazol-4-yl-, 1-(2-Cycloheptylethyl)-2-methyl-imidazol-4-yl-, 1-(3-Cyclopropylpropyl)-2-methyl-imidazol-4-yl-, 1-(3-Cyclobutylpropyl)-2-methyl-imidazol-4-yl-, 1-(3-Cyclopentylpropyl)-2-methyl-imidazol-4-yl-, 1-(3-Cyclohexylpropyl)-2-methyl-imidazol-4-yl, 1-(3-Cycloheptylpropyl)-2-methyl-imidazol-4-yl-, 1-(2,2,2-Trifluorethyl)-2-methyl-imidazol-4-yl-, 1-(3,3,3-Trifluorpropyl)-2-methyl-imidazol-4-yl-, 1-Benzyl-2-methyl-imidazol-4-yl-, 1-(2-Phenylethyl)-2-methyl-imidazol-4-yl, 1-(3-Phenylpropyl)-2-methyl-imidazol-4-yl-, 1-(4-Fluor-benzyl)-2-methyl-imidazol-4-yl-, 1-(4-Chlor-benzyl)-2-methyl-imidazol-4-yl-, 1-(3-Chlor-benzyl)-2-methyl-imidazol-4-yl-, 1-(4-Trifluormethyl-benzyl)-2-methyl-imidazol-4-yl-, 1-(3-Methyl-benzyl)-2-methyl-imidazol-4-yl-, 1-(4-Methyl-benzyl)-2-methyl-imidazol-4-yl-, 1-(3-Methoxy-benzyl)-2-methyl-imidazol-4-yl-, 1-(4-Methoxy-benzyl)-2-methyl-imidazol-4-yl-, 1-(3,4-Dimethoxy-benzyl)-2-methyl-imidazol-4-yl-, 1-(3,5-Dimethoxy-benzyl)-2-methyl-imidazol-4-yl-, 1-Carboxymethyl-imidazol-4-yl-, 1-(2-Carboxyethyl)-imidazol-4-yl-, 1-(3-Carboxypropyl)-imidazol-4-yl-, 1-(4-Carboxybutyl)-imidazol-4-yl-, 1-(5-Carboxypentyl)-imidazol-4-yl-, 1-(6-Carboxyhexyl)-imidazol-4-yl-, 1-(7-Carboxyheptyl)-imidazol-4-yl-, 1-Methoxycarbonylmethyl-imidazol-4-yl-, 1-(2-Methoxycarbonylethyl)-imidazol-4-yl-, 1-(3-Methoxycarbonylpropyl)-imidazol-4-yl-, 1-(4-Methoxycarbonylbutyl)-imidazol-4-yl-, 1-(5-Methoxycarbonylpentyl)-imidazol-4-yl-, 1-(6-Methoxycarbonylhexyl)-imidazol-4-yl-, 1-(7-Methoxycarbonylheptyl)-imidazol-4-yl-, 1-Ethoxycarbonylmethyl-imidazol-4-yl-, 1-(2-Ethoxycarbonylethyl)-imidazol-4-yl-, 1-(3-Ethoxycarbonylpropyl)-imidazol-4-yl-, 1-(4-Ethoxycarbonylbutyl)-imidazol-4-yl-, 1-(5-Ethoxycarbonylpentyl)-imidazol-4-yl-, 1-(6-Ethoxycarbonylhexyl)-imidazol-4-yl-, 1-(7-Ethoxycarbonylheptyl)-imidazol-4-yl-, 1-n-Propoxycarbonylmethyl-imidazol-4-yl-, 1-(2-n-Propoxycarbonylethyl)-imidazol-4-yl-, 1-(3-n-Propoxycarbonylpropyl)-imidazol-4-yl-, 1-(4-n-Propoxycarbonylbutyl)-imidazol-4-yl-, 1-(5-n-Propoxycarbonylpentyl)-imidazol-4-yl-, 1-(6-n-Propoxycarbonylhexyl)-imidazol-4-yl-, 1-(7-n-Propoxycarbonylheptyl)-imidazol-4-yl-, 1-Isopropoxycarbonylmethyl-imidazol-4-yl-, 1-(2-Isopropoxycarbonylethyl)-imidazol-4-yl-, 1-(3-Isopropoxycarbonylpropyl)-imidazol-4-yl-, 1-(4-Isopropoxycarbonylbutyl)-imidazol-4-yl-, 1-(5-Isopropoxycarbonylpentyl)-imidazol-4-yl-, 1-(6-Isopropoxycarbonylhexyl)-imidazol-4-yl-, 1-(7-Isopropoxycarbonylheptyl)-imidazol-4-yl-, 1-Aminocarbonylmethyl-imidazol-4-yl-, 1-(2-Aminocarbonylethyl)-imidazol-4-yl-, 1-(3-Aminocarbonylpropyl)-imidazol-4-yl-, 1-(4-Aminocarbonylbutyl)-imidazol-4-yl-, 1-(5-Aminocarbonylpentyl)-imidazol-4-yl-, 1-(6-Aminocarbonylhexyl)-imidazol-4-yl-, 1-(7-Aminocarbonylheptyl)-imidazol-4-yl-, 1-Methylaminocarbonylmethyl-imidazol-4-yl-, 1-(2-Methylaminocarbonylethyl)-imidazol-4-yl-, 1-(3-Methylaminocarbonylpropyl)-imidazol-4-yl-, 1-(4-Methylaminocarbonylbutyl)-imidazol-4-yl-, 1-(5-Methylaminocarbonylpentyl)-imidazol-4-yl-, 1-(6-Methylaminocarbonylhexyl)-imidazol-4-yl-, 1-(7-Methylaminocarbonylheptyl)-imidazol-4-yl-, 1-Ethylaminocarbonylmethyl-imidazol-4-yl-, 1-(2-Ethylaminocarbonylethyl)-imidazol-4-yl-, 1-(3-Ethylaminocarbonylpropyl)-imidazol-4-yl-, 1-(4-Ethylaminocarbonylbutyl)-imidazol-4-yl-, 1-(5-Ethylamino-

carbonylpentyl)-imidazol-4-yl-, 1-(6-Ethylaminocarbonylhexyl)-imidazol-4-yl-, 1-(7-Ethylaminocarbonylheptyl)-imidazol-4-yl-, 1-n-Propylaminocarbonylmethyl-imidazol-4-yl-, 1-(2-n-Propylaminocarbonylethyl)-imidazol-4-yl-, 1-(3-n-Propylaminocarbonylpropyl)-imidazol-4-yl-, 1-(4-n-Propylaminocarbonylbutyl)-imidazol-4-yl-, 1-(5-n-Propylaminocarbonylpentyl)-imidazol-4-yl-, 1-(6-n-Propylaminocarbonylhexyl)-imidazol-4-yl-, 1-(7-n-Propylaminocarbonylheptyl)-imidazol-4-yl-, 1-Isopropylaminocarbonylmethyl-imidazol-4-yl-, 1-(2-Isopropylaminocarbonylethyl)-imidazol-4-yl-, 1-(3-Isopropylaminocarbonylpropyl)-imidazol-4-yl-, 1-(4-Isopropylaminocarbonylbutyl)-imidazol-4-yl-, 1-(5-Isopropylaminocarbonylpentyl)-imidazol-4-yl-, 1-(6-Isopropylaminocarbonylhexyl)-imidazol-4-yl-, 1-(7-Isopropylaminocarbonylheptyl)-imidazol-4-yl-, 1-Dimethylaminocarbonylmethyl-imidazol-4-yl-, 1-(2-Dimethylaminocarbonylethyl)-imidazol-4-yl-, 1-(3-Dimethylaminocarbonylpropyl)-imidazol-4-yl-, 1-(4-Dimethylaminocarbonylbutyl)-imidazol-4-yl-, 1-(5-Dimethylaminocarbonylpentyl)-imidazol-4-yl-, 1-(6-Dimethylaminocarbonylhexyl)-imidazol-4-yl-, 1-(7-Dimethylaminocarbonylheptyl)-imidazol-4-yl-, 1-Diethylaminocarbonylmethyl-imidazol-4-yl-, 1-(2-Diethylaminocarbonylethyl)-imidazol-4-yl-, 1-(3-Diethylaminocarbonylpropyl)-imidazol-4-yl-, 1-(4-Diethylaminocarbonylbutyl)-imidazol-4-yl-, 1-(5-Diethylaminocarbonylpentyl)-imidazol-4-yl-, 1-(6-Diethylaminocarbonylhexyl)-imidazol-4-yl-, 1-(7-Diethylaminocarbonylheptyl)-imidazol-4-yl-, 1-Di-n-propylaminocarbonylmethyl-imidazol-4-yl-, 1-(2-Di-n-propylaminocarbonylethyl)-imidazol-4-yl-, 1-(3-Di-n-propylaminocarbonylpropyl)-imidazol-4-yl-, 1-(4-Di-n-propylaminocarbonylbutyl)-imidazol-4-yl-, 1-(5-Di-n-propylaminocarbonylpentyl)-imidazol-4-yl-, 1-(6-Di-n-propylaminocarbonylhexyl)-imidazol-4-yl-, 1-(7-Di-n-propylaminocarbonylheptyl)-imidazol-4-yl-, 1-Diisopropylaminocarbonylmethyl-imidazol-4-yl-, 1-(2-Diisopropylaminocarbonylethyl)-imidazol-4-yl-, 1-(3-Diisopropylaminocarbonylpropyl)-imidazol-4-yl-, 1-(4-Diisopropylaminocarbonylbutyl)-imidazol-4-yl-, 1-(5-Diisopropylaminocarbonylpentyl)-imidazol-4-yl-, 1-(6-Diisopropylaminocarbonylhexyl)-imidazol-4-yl-, 1-(7-Diisopropylaminocarbonylheptyl)-imidazol-4-yl-, 1-Morpholinocarbonylmethyl-imidazol-4-yl-, 1-(2-Morpholinocarbonylethyl)-imidazol-4-yl-, 1-(3-Morpholinocarbonylpropyl)-imidazol-4-yl-, 1-(4-Morpholinocarbonylbutyl)-imidazol-4-yl-, 1-(5-Morpholinocarbonylpentyl)-imidazol-4-yl-, 1-(6-Morpholinocarbonylhexyl)-imidazol-4-yl-, 1-(7-Morpholinocarbonylheptyl)-imidazol-4-yl-, 1-Thiomorpholinocarbonylmethyl-imidazol-4-yl-, 1-(2-Thiomorpholinocarbonylethyl)-imidazol-4-yl-, 1-(3-Thiomorpholinocarbonylpropyl)-imidazol-4-yl-, 1-(4-Thiomorpholinocarbonylbutyl)-imidazol-4-yl-, 1-(5-Thiomorpholinocarbonylpentyl)-imidazol-4-yl-, 1-(6-Thiomorpholinocarbonylhexyl)-imidazol-4-yl-, 1-(7-Thiomorpholinocarbonylheptyl)-imidazol-4-yl-, 1-Oxidothiomorpholinocarbonylmethyl-imidazol-4-yl-, 1-(2-Oxidothiomorpholinocarbonylethyl)-imidazol-4-yl-, 1-(3-Oxidothiomorpholinocarbonylpropyl)-imidazol-4-yl-, 1-(4-Oxidothiomorpholinocarbonylbutyl)-imidazol-4-yl-, 1-(5-Oxidothiomorpholinocarbonylpentyl)-imidazol-4-yl-, 1-(6-Oxidothiomorpholinocarbonylhexyl)-imidazol-4-yl-, 1-(7-Oxidothiomorpholinocarbonylheptyl)-imidazol-4-yl-, 1-Carboxymethyl-2-methyl-imidazol-4-yl-, 1-(2-Carboxyethyl)-2-methyl-imidazol-4-yl-, 1-(3-Carboxypropyl)-2-methyl-imidazol-4-yl-, 1-(4-Carboxybutyl)-2-methyl-imidazol-4-yl-, 1-(5-Carboxypentyl)-2-methyl-imidazol-4-yl-, 1-(6-Carboxyhexyl)-2-methyl-imidazol-4-yl-, 1-(7-Carboxyheptyl)-2-methyl-imidazol-4-yl-, 1-Methoxycarbonylmethyl-2-methyl-imidazol-4-yl-, 1-(2-Methoxycarbonylethyl)-2-methyl-imidazol-4-yl-, 1-(3-Methoxycarbonylpropyl)-2-methyl-imidazol-4-yl-, 1-(4-Methoxycarbonylbutyl)-2-methyl-imidazol-4-yl-, 1-(5-Methoxycarbonylpentyl)-2-methyl-imidazol-4-yl-, 1-(6-Methoxycarbonylhexyl)-2-methyl-imidazol-4-yl-, 1-(7-Methoxycarbonylheptyl)-2-methyl-imidazol-4-yl-, 1-Ethoxycarbonylmethyl-2-methyl-imidazol-4-yl-, 1-(2-Ethoxycarbonylethyl)-2-methyl-imidazol-4-yl-, 1-(3-Ethoxycarbonylpropyl)-2-methyl-imidazol-4-yl-, 1-(4-Ethoxycarbonylbutyl)-2-methyl-imidazol-4-yl-, 1-(5-Ethoxycarbonylpentyl)-2-methyl-imidazol-4-yl-, 1-(6-Ethoxycarbonylhexyl)-2-methyl-imidazol-4-yl-, 1-(7-Ethoxycarbonylheptyl)-2-methyl-imidazol-4-yl-, 1-n-Propoxycarbonylmethyl-2-methyl-imidazol-4-yl-, 1-(2-n-Propoxycarbonylethyl)-2-methyl-imidazol-4-yl-, 1-(3-n-Propoxycarbonylpropyl)-2-methyl-imidazol-4-yl-, 1-(4-n-Propoxycarbonylbutyl)-2-methyl-imidazol-4-yl-, 1-(5-n-Propoxycarbonylpentyl)-2-methyl-imidazol-4-yl-, 1-(6-n-Propoxycarbonylhexyl)-2-methyl-imidazol-4-yl-, 1-(7-n-Propoxycarbonylheptyl)-2-methyl-imidazol-4-yl-, 1-Isopropoxycarbonylmethyl-2-methyl-imidazol-4-yl-, 1-(2-Isopropoxycarbonylethyl)-2-methyl-imidazol-4-yl-, 1-(3-Isopropoxycarbonylpropyl)-2-methyl-imidazol-4-yl-, 1-(4-Isopropoxycarbonylbutyl)-2-methyl-imidazol-4-yl-, 1-(5-Isopropoxycarbonylpentyl)-2-methyl-imidazol-4-yl-, 1-(6-Isopropoxycarbonylhexyl)-2-methyl-imidazol-4-yl-, 1-(7-Isopropoxycarbonylheptyl)-2-methyl-imidazol-4-yl-, 1-Aminocarbonylmethyl-2-methyl-imidazol-4-yl-, 1-(2-Aminocarbonylethyl)-2-methyl-imidazol-4-yl-, 1-(3-Aminocarbonylpropyl)-2-methyl-imidazol-4-yl-, 1-(4-Aminocarbonylbutyl)-2-methyl-imidazol-4-yl-, 1-(5-Aminocarbonylpentyl)-2-methyl-imidazol-4-yl-, 1-(6-Aminocarbonylhexyl)-2-methyl-imidazol-4-yl-, 1-(7-Aminocarbonylheptyl)-2-methyl-imidazol-4-yl-, 1-Methylaminocarbonylmethyl-2-methyl-imidazol-4-yl-, 1-(2-Methylaminocarbonylethyl)-2-methyl-imidazol-4-yl-, 1-(3-Methylaminocarbonylpropyl)-2-methyl-imidazol-4-yl-, 1-(4-Methylaminocarbonylbutyl)-2-methyl-imidazol-4-yl-, 1-(5-Methylaminocarbonylpentyl)-2-methyl-imidazol-4-yl-, 1-(6-Methylaminocarbonylhexyl)-2-methyl-imidazol-4-yl-, 1-(7-Methylaminocarbonylheptyl)-2-methyl-imidazol-4-yl-, 1-Ethylaminocarbonylmethyl-2-methyl-imidazol-4-yl-, 1-(2-Ethylaminocarbonylethyl)-2-methyl-imidazol-4-yl-, 1-(3-Ethylaminocarbonylpropyl)-2-methyl-imidazol-4-yl-, 1-(4-Ethylaminocarbonylbutyl)-2-methyl-imidazol-4-yl-,

1-(5-Ethylaminocarbonylpentyl)-2-methyl-imidazol-4-yl-, 1-(6-Ethylaminocarbonylhexyl)-2-methyl-imidazol-4-yl-, 1-(7-Ethylaminocarbonylheptyl)-2-methyl-imidazol-4-yl-, 1-n-Propylaminocarbonylmethyl-2-methyl-imidazol-4-yl-, 1-(2-n-Propylaminocarbonylethyl)-2-methyl-imidazol-4-yl-, 1-(3-n-Propylaminocarbonylpropyl)-2-methyl-imidazol-4-yl-, 1-(4-n-Propylaminocarbonylbutyl)-2-methyl-imidazol-4-yl-, 1-(5-n-Propylaminocarbonylpentyl)-2-methyl-imidazol-4-yl-, 1-(6-n-Propylaminocarbonylhexyl)-2-methyl-imidazol-4-yl-, 1-(7-n-Propylaminocarbonylheptyl)-2-methyl-imidazol-4-yl-, 1-Isopropylaminocarbonylmethyl-2-methyl-imidazol-4-yl-, 1-(2-Isopropylaminocarbonylethyl)-2-methyl-imidazol-4-yl-, 1-(3-Isopropylaminocarbonylpropyl)-2-methyl-imidazol-4-yl-, 1-(4-Isopropylaminocarbonylbutyl)-2-methyl-imidazol-4-yl-, 1-(5-Isopropylaminocarbonylpentyl)-2-methyl-imidazol-4-yl-, 1-(6-Isopropylaminocarbonylhexyl)-2-methyl-imidazol-4-yl-, 1-(7-Isopropylaminocarbonylheptyl)-2-methyl-imidazol-4-yl-, 1-Dimethylaminocarbonylmethyl-2-methyl-imidazol-4-yl-, 1-(2-Dimethylaminocarbonylethyl)-2-methyl-imidazol-4-yl-, 1-(3-Dimethylaminocarbonylpropyl)-2-methyl-imidazol-4-yl-, 1-(4-Dimethylaminocarbonylbutyl)-2-methyl-imidazol-4-yl-, 1-(5-Dimethylaminocarbonylpentyl)-2-methyl-imidazol-4-yl-, 1-(6-Dimethylaminocarbonylhexyl)-2-methyl-imidazol-4-yl-, 1-(7-Dimethylaminocarbonylheptyl)-2-methyl-imidazol-4-yl-, 1-Diethylaminocarbonylmethyl-2-methyl-imidazol-4-yl-, 1-(2-Diethylaminocarbonylethyl)-2-methyl-imidazol-4-yl-, 1-(3-Diethylaminocarbonylpropyl)-2-methyl-imidazol-4-yl-, 1-(4-Diethylaminocarbonylbutyl)-2-methyl-imidazol-4-yl, 1-(5-Diethylaminocarbonylpentyl)-2-methyl-imidazol-4-yl-, 1-(6-Diethylaminocarbonylhexyl)-2-methyl-imidazol-4-yl-, 1-(7-Diethylaminocarbonylheptyl)-2-methyl-imidazol-4-yl-, 1-Di-n-propylaminocarbonylmethyl-2-methyl-imidazol-4-yl-, 1-(2-Di-n-propylaminocarbonylethyl)-2-methyl-imidazol-4-yl-, 1-(3-Di-n-propylaminocarbonylpropyl)-2-methyl-imidazol-4-yl-, 1-(4-Di-n-propylaminocarbonylbutyl)-2-methyl-imidazol-4-yl-, 1-(5-Di-n-propylaminocarbonylpentyl)-2-methyl-imidazol-4-yl-, 1-(6-Di-n-propylaminocarbonylhexyl)-2-methyl-imidazol-4-yl-, 1-(7-Di-n-propylaminocarbonylheptyl)-2-methyl-imidazol-4-yl-, 1-Diisopropylaminocarbonylmethyl-2-methyl-imidazol-4-yl-, 1-(2-Diisopropylaminocarbonylethyl)-2-methyl-imidazol-4-yl-, 1-(3-Diisopropylaminocarbonylpropyl)-2-methyl-imidazol-4-yl-, 1-(4-Diisopropylaminocarbonylbutyl)-2-methyl-imidazol-4-yl-, 1-(5-Diisopropylaminocarbonylpentyl)-2-methyl-imidazol-4-yl-, 1-(6-Diisopropylaminocarbonylhexyl)-2-methyl-imidazol-4-yl-, 1-(7-Diisopropylaminocarbonylheptyl)-2-methyl-imidazol-4-yl-, 1-Morpholinocarbonylmethyl-2-methyl-imidazol-4-yl-, 1-(2-Morpholinocarbonylethyl)-2-methyl-imidazol-4-yl-, 1-(3-Morpholinocarbonylpropyl)-2-methyl-imidazol-4-yl-, 1-(4-Morpholinocarbonylbutyl)-2-methyl-imidazol-4-yl-, 1-(5-Morpholinocarbonylpentyl)-2-methyl-imidazol-4-yl-, 1-(6-Morpholinocarbonylhexyl)-2-methyl-imidazol-4-yl-, 1-(7-Morpholinocarbonylheptyl)-2-methyl-imidazol-4-yl-, 1-Thiomorpholinocarbonylmethyl-2-methyl-imidazol-4-yl-, 1-(2-Thiomorpholinocarbonylethyl)-2-methyl-imidazol-4-yl-, 1-(3-Thiomorpholinocarbonylpropyl)-2-methyl-imidazol-4-yl-, 1-(4-Thiomorpholinocarbonylbutyl)-2-methyl-imidazol-4-yl-, 1-(5-Thiomorpholinocarbonylpentyl)-2-methyl-imidazol-4-yl-, 1-(6-Thiomorpholinocarbonylhexyl)-2-methyl-imidazol-4-yl-, 1-(7-Thiomorpholinocarbonylheptyl)-2-methyl-imidazol-4-yl-, 1-Oxidothiomorpholinocarbonylmethyl-2-methyl-imidazol-4-yl-, 1-(2-Oxidothiomorpholinocarbonylethyl)-2-methyl-imidazol-4-yl-, 1-(3-Oxidothiomorpholinocarbonylpropyl)-2-methyl-imidazol-4-yl-, 1-(4-Oxidothiomorpholinocarbonylbutyl)-2-methyl-imidazol-4-yl-, 1-(5-Oxidothiomorpholinocarbonylpentyl)-2-methyl-imidazol-4-yl-, 1-(6-Oxidothiomorpholinocarbonylhexyl)-2-methyl-imidazol-4-yl-, 1-(7-Oxidothiomorpholinocarbonylheptyl)-2-methyl-imidazol-4-yl-, 1-(2-Hydroxyethyl)-imidazol-4-yl-, 1-(3-Hydroxypropyl)-imidazol-4-yl-, 1-(4-Hydroxybutyl)-imidazol-4-yl-, 1-(2-Methoxyethyl)-imidazol-4-yl-, 1-(3-Methoxypropyl)-imidazol-4-yl-, 1-(4-Methoxybutyl)-imidazol-4-yl-, 1-(2-Ethoxyethyl)-imidazol-4-yl-, 1-(3-Ethoxypropyl)-imidazol-4-yl-, 1-(4-Ethoxybutyl)-imidazol-4-yl-, 1-(2-n-Propoxyethyl)-imidazol-4-yl-, 1-(3-n-Propoxypropyl)-imidazol-4-yl-, 1-(4-n-Propoxybutyl)-imidazol-4-yl-, 1-(2-Isopropoxyethyl)-imidazol-4-yl-, 1-(3-Isopropoxypropyl)-imidazol-4-yl-, 1-(4-Isopropoxybutyl)-imidazol-4-yl-, 1-(2-Imidazol-1-yl-ethyl)-imidazol-4-yl-, 1-(3-Imidazol-1-yl-propyl)-imidazol-4-yl-, 1-(4-Imidazol-1-yl-butyl)-imidazol-4-yl-, 1-(2,2-Diphenyl-ethyl)-imidazol-4-yl-, 1-(3,3-Diphenyl-propyl)-imidazol-4-yl-, 1-(4,4-Diphenyl-butyl)-imidazol-4-yl-, 1-(2-Hydroxyethyl)-2-methyl-imidazol-4-yl-, 1-(3-Hydroxypropyl)-2-methyl-imidazol-4-yl-, 1-(4-Hydroxybutyl)-2-methyl-imidazol-4-yl-, 1-(2-Methoxyethyl)-2-methyl-imidazol-4-yl-, 1-(3-Methoxypropyl)-2-methyl-imidazol-4-yl-, 1-(4-Methoxybutyl)-2-methyl-imidazol-4-yl-, 1-(2-Ethoxyethyl)-2-methyl-imidazol-4-yl-, 1-(3-Ethoxypropyl)-2-methyl-imidazol-4-yl-, 1-(4-Ethoxybutyl)-2-methyl-imidazol-4-yl-, 1-(2-n-Propoxyethyl)-2-methyl-imidazol-4-yl-, 1-(3-n-Propoxypropyl)-2-methyl-imidazol-4-yl-, 1-(4-n-Propoxybutyl)-2-methyl-imidazol-4-yl-, 1-(2-Isopropoxyethyl)-2-methyl-imidazol-4-yl, 1-(3-Isopropoxypropyl)-2-methyl-imidazol-4-yl-, 1-(4-Isopropoxybutyl)-2-methyl-imidazol-4-yl-, 1-(2-Imidazol-1-yl-ethyl)-2-methyl-imidazol-4-yl-, 1-(3-Imidazol-1-yl-propyl)-2-methyl-imidazol-4-yl-, 1-(4-Imidazol-1-yl-butyl)-2-methyl-imidazol-4-yl-, 1-(2,2-Diphenyl-ethyl)-2-methyl-imidazol-4-yl-, 1-(3,3-Diphenyl-propyl)-2-methyl-imidazol-4-yl-, 1-(4,4-Diphenyl-butyl)-2-methyl-imidazol-4-yl-, Benzimidazol-2-yl-, 1-Methylbenzimidazol-2-yl-, 1-Ethylbenzimidazol-2-yl-, 1-n-Propylbenzimidazol-2-yl-, 1-Isopropylbenzimidazol-2-yl-, 1-n-Butylbenzimidazol-2-yl-, 1-Isobutylbenzimidazol-2-yl-, 1-n-Pentylbenzimidazol-2-yl-, 1-n-Hexylbenzimidazol-2-yl-, 1-Cyclopropylbenzimidazol-2-yl-, 1-Cyclobutylbenzimidazol-2-yl-, 1-Cyclopentylbenzimidazol-2-yl-, 1-Cyclohexylbenzimidazol-2-yl-, 1,5-Dimethyl-benzimidazol-2-yl-, 1,6-Dimethyl-benzimidazol-2-yl-, 1,4-Dimethyl-benzimidazol-2-yl-,5-Fluor-1-methyl-benzimidazol-2-yl-, 6-Fluor-1-methyl-benzimidazol-2-yl-, 5-Trifluormethyl-benzimidazol-2-yl-, 5-Trifluormethyl-1-methyl-benzimidazol-2-yl-, Imidazo[1,2-a]pyridin-2-yl-, 5,6,7,8-Tetrahydro-imidazo[1,2-a]pyrimidin-2-yl-, Imidazo[1,2-a]pyrimidin-2-yl-, Imidazo[4,5-b]pyridin-2-yl-, Imidazo[4,5-c]pyridin-2-yl-, Imidazo[2,1-b]thiazol-6-yl-, Imidazo[1,2-c]pyrimidin-2-yl-, Imidazo[1,2-a]pyrazin-2-yl-, Imidazo[1,2-b]pyidazin-2-yl-, Imidazo[4,5-c]pyridin-2-yl-, Purin-8-yl-, Imidazo[4,5-b]pyrazin-2-yl-, Imidazo[4,5-c]pyridazin-2-yl-, Imidazo[4,5-d]pyridazin-2-yl-,4,5-Dihydro-2H-pyridazin-3-on-6-yl-, 2-Methyl-4,5-dihydro-2H-pyridazin-3-on-6-yl-, 2-Benzyl-4,5-dihydro-2H-pyridazin-3-on-6-yl-, 2H-Pyridazin-3-on-6-yl-, 2-Methyl-pyridazin-3-on-6-yl-, 2-Benzyl-pyridazin-3-on-6-yl-, Aminocarbonylamino-, Methylaminocarbonylamino-, Dimethylaminocarbonylamino-, N-Methylaminocarbonyl-methylamino-, N-(Dimethylaminocarbonyl)-methylamino-,N-Dimethylaminocarbonyl-ethylamino-, N-Dimethylaminocarbonylisopropylamino-, N-(Dimethylaminocarbonyl)-n-pentylamino-, N-Methylaminocarbonyl-ethylamino-, N-Methylaminocarbonyl-n-pentylamino-, N-Methylaminocarbonyl-cyclohexylamino-, Ethylaminocarbonylamino-, N-Ethylaminocarbonyl-methylamino-, N-Ethylaminocarbonyl-ethylamino-, N-Ethylaminocarbonylcyclohexylamino-, Diethylaminocarbonylamino-, N-(Diethylaminocarbonyl)-methylamino-, N-(Diethylaminocarbonyl)-ethylamino-,N-(Diethylaminocarbonyl)-n-butylamino-, Isopropylaminocarbonylamino-, N-Isopropylaminocarbonyl-methylamino-, n-Butylaminocarbonylamino-, N-(n-Butylaminocarbonyl)-methylamino-, N-(n-Butylaminocarbonyl)-ethylamino-, N-(n-Butylaminocarbonyl)-isopropylamino-, N-(n-Butylaminocarbonyl)-n-butylamino-, N-(n-Butylaminocarbonyl)-cyclohexylamino-, N-(Di-(n-butyl)-aminocarbonyl)-amino-, N-(Di-(n-butyl)-aminocarbonyl)-methylamino-, N-(Di-(n-butyl)-aminocarbonyl)-ethylamino-, N-(Di-(n-butyl)-aminocarbonyl)-n-butylamino-, N-(n-Pentylaminocarbonyl)-methylamino-, N-(n-Pentylaminocarbonyl)-ethyl-amino-, N-(n-Hexylaminocarbonyl)-ethylamino-, n-Hexylaminocarbonylamino-, N-(n-Hexylaminocarbonyl)-n-butylamino-, N-(n-Hexylaminocarbonyl)-n-pentylamino-, N-(n-Hexylaminocarbonyl)-cyclohexylamino-, Di-(n-hexyl)-aminocarbonylamino-, N-(Di-(n-hexyl)-aminocarbonyl)-methylamino-, N-((n-hexyl)-methylaminocarbonyl)-amino-, Cyclohexylaminocarbonylamino-,N-Cyclohexylaminocarbonyl-methylamino-, N-Cyclohexylaminocarbonyl-ethylamino-, N-Cyclohexylaminocarbonyl-n-butylamino-, N-Cyclohexylaminocarbonyl-isobutylamino-, N-Cyclohexylaminocarbonyl-n-pentylamino-, N-Cyclohexylaminocarbonyl-cyclohexylamino-, N-(Ethyl-cyclohexylaminocarbonyl)-methylamino-, N-(Propyl-cyclohexylaminocarbonyl)-methylamino-, N-(n-Butyl-cyclohexylaminocarbonyl)-methylamino-, Allylaminocarbonylamino-,Benzylaminocarbonylamino-, N-Benzylaminocarbonyl-isobutylamino-, Phenylaminocarbonylamino-, Pyrrolidinocarbonylamino-, Pyrrolidinocarbonylmethylamino-, Piperidinocarbonylamino-, Hexamethyleniminocarbonylamino-, Morpholinocarbonylamino-, 3,4,5,6-Tetrahydro-2-pyrimidon-1-yl-, 3-Methyl-3,4,5,6-tetrahydro-2-pyrimidon-1-yl-, 3-Ethyl-3,4,5,6-tetrahydro-2-pyrimidon-1-yl-, 3-n-Propyl-3,4,5,6-tetrahydro-2-pyrimidon-1-yl-, 3-Isopropyl-3,4,5,6-tetrahydro-2-pyrimidon-1-yl-, Carboxy-, Aminocarbonyl-, Methylaminocarbonyl-, Ethylaminocarbonyl-, n-Propylaminocarbonyl-, Isopropylaminocarbonyl-, Dimethylaminocarbonyl-, Diethylaminocarbonyl-, Di-n-propylaminocarbonyl-, Diisopropylaminocarbonyl-, N-Methyl-ethylaminocarbonyl- oder N-Ethyl-isopropylaminocarbonylgruppe,

sowie für die bei der Definition der Reste $R_2$ und $R_b$ eingangs erwähnte Bedeutung der in vivo in eine Carboxygruppe überführbare Gruppe beispielsweise jeweils zusätzlich die Bedeutung der Methoxycarbonyl-, Ethoxycarbonyl-, n-Propyloxycarbonyl-, Isopropyloxycarbonyl-, n-Butyloxycarbonyl-, Isobutyloxycarbonyl-, tert.Butyloxycarbonyl-, n-Pentyloxycarbonyl-, Isoamyloxycarbonyl-, n-Hexyloxycarbonyl-, Cyclopentyloxycarbonyl-, Cyclohexyloxycarbonyl-, Benzyloxycarbonyl-, 1-Phenylethyloxycarbonyl-, 2-Phenylethyloxycarbonyl-, 3-Phenylpropyloxycarbonyl-, Methoxymethoxycarbonyl-, Cinnamyloxycarbonyl-, Acetoxymethoxycarbonyl-, Propionyloxymethoxycarbonyl-, n-Butyryloxymethoxycarbonyl-, Isobutyryloxymethoxycarbonyl-, n-Pentanoyloxymethoxycarbonyl-, Isopentanoyloxymethoxycarbonyl-, Pivaloyloxymethoxycarbonyl-, n-Hexanoyloxymethoxycarbonyl-, Cyclopentanoyloxymethoxycarbonyl-, Cyclohexanoyloxymethoxycarbonyl-, Phenylacetoxymethoxycarbonyl-, 2-Phenylpropionyloxymethoxycarbonyl-, 3-Phenylpropionyloxymethoxycarbonyl-, 4-Phenylbutyryloxymethoxycarbonyl-, Benzoyloxymethoxycarbonyl-, 1-Acetoxyethoxycarbonyl-, 1-Propionyloxyethoxycarbonyl-, 1-n-Butyryloxyethoxycarbonyl-, 1-Isobutyryloxyethoxycarbonyl-, 1-n-Pentanoyloxyethoxycarbonyl-, 1-Isopentanoyloxyethoxycarbonyl-, 1-Pivaloyloxyethoxycarbonyl-, 1-n-Hexanoyloxyethoxycarbonyl-, 1-Cyclopentanoyloxyethoxycarbonyl-, 1-Cyclohexanoyloxyethoxycarbonyl-, 1-Phenylacetoxyethoxycarbonyl-, 1-(1-Phenylpropionyloxy)-ethoxycarbonyl-, 1-(2-Phenylpropionyloxy)-ethoxycarbonyl-, 1-(3-Phenylbutyryloxy)-ethoxycarbonyl-, 1-Benzoyloxyethoxycarbonyl-, Methoxycarbonyloxymethoxycarbonyl-, Ethoxycarbonyloxymethoxycarbonyl-, n-Propyloxycarbonyloxymethoxycarbonyl-, Isopropyloxycarbonyloxymethoxycarbonyl-,n-Butyloxycarbonyloxymethoxycarbonyl-, Isobutyloxycarbonyloxymethoxycarbonyl-, tert.Butyloxycarbonyloxymethoxycarbonyl-,n-Pentyloxycarbonyloxymethoxycarbonyl-, Isoamyloxycarbonyloxymethoxycarbonyl-, n-Hexyloxycarbonyloxymethoxycarbonyl-,

Cyclopentyloxycarbonyloxymethoxycarbonyl-,Cyclohexyloxycarbonyloxymethoxycarbonyl-, Benzyloxycarbonyloxymethoxycarbonyl-, 1-Phenylethoxycarbonyloxymethoxycarbonyl-,2-Phenylethoxycarbonyloxymethoxycarbonyl-, 3-Phenylpropyloxycarbonyloxymethoxycarbonyl-, Cinnamyloxycarbonyloxymethoxycarbonyl-, 1-(Methoxycarbonyloxy)-ethoxycarbonyl-,1-(Ethoxycarbonyloxy)-ethoxycarbonyl-, 1-(n-Propyloxycarbonyloxy)-ethoxycarbonyl-, 1-(Isopropyloxycarbonyloxy)-ethoxycarbonyl-, 1-(n-Butyloxycarbonyloxy)-ethoxycarbonyl-, 1-(Isobutyloxycarbonyloxy)-ethoxycarbonyl-, 1-(tert.Butyloxycarbonyloxy)-ethoxycarbonyl-, 1-(n-Pentyloxycarbonyloxy)-ethoxycarbonyl-, 1-(Isoamyloxycarbonyloxy)-ethoxycarbonyl-, 1-(n-Hexyloxycarbonyloxy)-ethoxycarbonyl-, 1-(Cyclopentyloxycarbonyloxy)-ethoxycarbonyl-, 1-(Cyclohexyloxycarbonyloxy)-ethoxycarbonyl-, 1-(Benzyloxycarbonyloxy)-ethoxycarbonyl-, 1-(1-Phenylethoxycarbonyloxy)-ethoxycarbonyl-, 1-(2-Phenylethoxycarbonyloxy)-ethoxycarbonyl-, 1-(3-Phenylpropyloxycarbonyloxy)-ethoxycarbonyl- oder 1-(Cinnamyloxycarbonyloxy)-ethoxycarbonylgruppe in Betracht.

Bevorzugte Verbindungen der obigen allgemeinen Formel I sind diejenigen, in denen
n die Zahl 0, 1, 2 oder 3 und
X eine Bindung oder
n die Zahl 1, 2 oder 3 und
X ein Sauerstoffatom,
A eine 1,4-Butadienylengruppe, die durch die Reste $R_1$ und $R_2$ substituiert ist und in der zusätzlich die Methingruppe in Position 7 des so gebildeten Benzimidazols durch ein Stickstoffatom ersetzt sein kann, wobei
$R_1$ ein Wasserstoffatom oder in 4-Stellung ein Fluor-, Chlor- oder Bromatom, eine Trifluormethylgruppe oder eine Alkylgruppe mit 1 bis 3 Kohlenstoffatomen und
$R_2$ ein Wasserstoffatom,
eine Alkylgruppe mit 1 bis 3 Kohlenstoffatomen,
in 6-Stellung eine Alkanoylaminogruppe mit 2 bis 5 Kohlenstoffatomen im Alkanoylteil oder eine Benzolsulfonylaminogruppe, welche beide am Stickstoffatom durch eine Alkylgruppe mit 1 bis 3 Kohlenstoffatomen substituiert sein können,
in 6-Stellung eine Phthalimino- oder Homophthaliminogruppe, wobei eine Carbonylgruppe in einer Phthaliminogruppe durch eine Methylengruppe ersetzt sein kann,
in 6-Stellung eine 5-, 6- oder 7-gliedrige Alkyleniminogruppe, in welcher eine Methylengruppe durch eine Carbonyl- oder Sulfonylgruppe ersetzt ist,
in 6-Stellung eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,
in 6-Stellung eine gegebenenfalls in 1-Stellung durch eine Alkylgruppe mit 1 bis 6 Kohlenstoffatomen oder eine Cycloalkylgruppe mit 3 bis 7 Kohlenstoffatomen substituierte Benzimidazol-2-yl-gruppe, wobei der Phenylkern in einer vorstehend erwähnten Benzimidazol-2-ylgruppe zusätzlich durch ein Fluoratom, eine Methyl- oder Trifluormethylgruppe substituiert sein kann, eine Imidazo[2,1-b]thiazol-6-yl-, Imidazo[1,2-a]pyridin-2-yl-, 5,6,7,8-Tetrahydro-imidazo[1,2-a]pyridin-2-yl-, Imidazo[1,2-a]pyrimidin-2-yl- oder Imidazo[4,5-b]pyridin-2-yl-gruppe,
in 6-Stellung eine Imidazol-4-yl-Gruppe, die in 1-Stellung durch eine Alkylgruppe mit 1 bis 7 Kohlenstoffatomen, die in 2-, 3-, 4-, 5-, 6- oder 7-Stellung durch eine Carboxy-, Alkoxycarbonyl-, Aminocarbonyl-, Alkylaminocarbonyl-, Dialkylaminocarbonyl-, Morpholinocarbonyl-, Thiomorpholinocarbonyl- oder 1-Oxido-thiomorpholinocarbonylgruppe substituiert sein kann, durch eine Alkylgruppe mit 1 bis 4 Kohlenstoffatomen, die in 2-, 3- oder 4-Stellung durch eine Hydroxy-, Alkoxy- oder Imidazol-1-yl-Gruppe substituiert ist, durch eine Alkylgruppe, die durch eine Trifluormethylgruppe, durch eine Cycloalkylgruppe mit 3 bis 7 Kohlenstoffatomen oder durch eine gegebenenfalls durch Fluor- oder Chloratome, durch Trifluormethyl-, Methyl- oder Methoxygruppen mono- oder disubstituierte Phenylgruppe substituiert ist, durch eine durch zwei Phenylgruppen substituierte Alkylgruppe oder durch eine Cycloalkylgruppe mit 3 bis 7 Kohlenstoffatomen substituiert ist, wobei, soweit nichts anderes erwähnt wurde, die vorstehend erwähnten Alkyl- und Alkoxyteile jeweils 1 bis 3 Kohlenstoffatome enthalten können,
in 7-Stellung eine in vivo in eine Carboxygruppe überführbare Gruppe,
in 7-Stellung eine Carbonylgruppe, die durch eine Hydroxygruppe, durch eine Alkoxygruppe mit 1 bis 6 Kohlenstoffatomen, wobei die Methoxygruppe zusätzlich durch eine Alkanoyloxy-, Alkoxycarbonyloxy- oder Cycloalkoxycarbonyloxygruppe substituiert ist, oder durch eine gegebenenfalls durch Alkylgruppen mono- oder disubsituierte Aminogruppe substituiert ist, wobei in den vorstehend erwähnten Gruppen der Alkanoylteil 2 oder 3 Kohlenstoffatome, der Alkyl- und Alkoxyteil jeweils 1 bis 6 Kohlenstoffatome und der Cycloalkoxyteil 5 bis 7 Kohlenstoffatome enthalten kann, oder

in 6-Stellung eine $R_5$-$NR_4$-CO-$NR_3$-Gruppe, in der

$R_3$ ein Wasserstoffatom, eine Alkylgruppe mit 1 bis 5 Kohlenstoffatomen, eine Cyclohexyl- oder Benzylgruppe,

$R_4$ ein Wasserstoffatom, eine Alkylgruppe mit 1 bis 6 Kohlenstoffatomen, eine Allyl-, Cyclohexyl-, Benzyl- oder Phenylgruppe,

$R_5$ ein Wasserstoffatom oder eine Alkylgruppe mit 1 bis 3 Kohlenstoffatomen oder

$R_4$ und $R_5$ zusammen mit dem dazwischen liegenden Stickstoffatom eine unverzweigte Cycloalkyleniminogruppe mit 4 bis 6 Kohlenstoffatomen oder eine Morpholinogruppe oder

$R_3$ und $R_4$ zusammen eine Alkylengruppe mit 2 oder 3 Kohlenstoffatomen darstellen,

$R_a$ eine Alkylgruppe mit 2 bis 4 Kohlenstoffatomen, eine Cycloalkylgruppe mit 3 oder 4 Kohlenstoffatomen oder eine Alkoxygruppe mit 2 oder 3 Kohlenstoffatomen,

$R_b$ eine in vivo in eine Carboxygruppe überführbare Gruppe, eine Carboxy- oder 1H-Tetrazolylgruppe und

$R_c$ ein Wasserstoffatom bedeuten,

deren Gemische von Stellungsisomeren und deren Salze.

Besonders bevorzugte Verbindungen der obigen allgemeinen Formel I sind diejenigen, in denen

n die Zahl 0, 1, 2 oder 3 und

X eine Bindung,

A eine 1,4-Butadienylengruppe, die durch die Reste $R_1$ und $R_2$ substituiert ist und in der zusätzlich die Methingruppe in Position 7 des so gebildeten Benzimidazols durch ein Stickstoffatom ersetzt sein kann, wobei

$R_1$ ein Wasserstoffatom oder in 4-Stellung eine Methylgruppe und

$R_2$ in 6-Stellung eine 1-Isopropyl-imidazol-4-yl- oder 1-Methyl-benzimidazol-2-yl-Gruppe oder in 7-Stellung eine in vivo in eine Carboxygruppe überführbare Gruppe oder eine Carboxygruppe darstellen,

$R_a$ eine n-Alkylgruppe mit 2 bis 4 Kohlenstoffatomen,

$R_b$ eine in vivo in eine Carboxygruppe überführbare Gruppe, eine Carboxy- oder 1H-Tetrazolylgruppe und

$R_c$ ein Wasserstoffatom bedeuten,

deren Gemische von Stellungsisomeren und deren Salze.

Erfindungsgemäß erhält man die erfindungsgemäßen Verbindungen nach folgenden Verfahren:

a) Umsetzung eines Benzimidazols der allgemeinen Formel

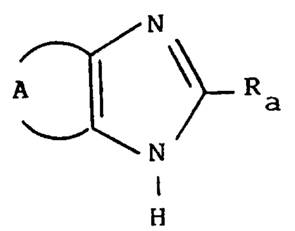

,(II)

in der

A und $R_a$ wie eingangs definiert sind, mit einer Biphenylverbindung der allgemeinen Formel

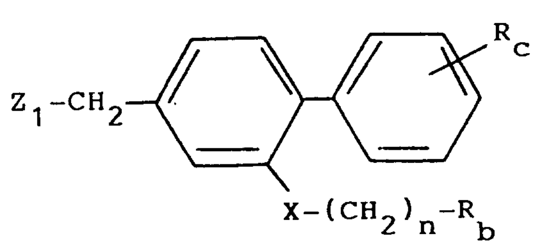

,(III)

in der

n, X, $R_b$ und $R_c$ wie eingangs definiert sind und $Z_1$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe, darstellt, und gegebenenfalls anschließende Hydrolyse.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid oder Benzol

gegebenenfalls in Gegenwart eines säurebindenden Mittels, wie Natriumcarbonat, Kaliumcarbonat, Natriumhydroxid, Natriumhydrid, Kalium-tert.butylat, Triethylamin oder Pyridin, wobei die beiden letzteren gleichzeitig auch als Lösungsmittel verwendet werden können, vorzugsweise bei Temperaturen zwischen 0 und 100 °C, z.B. bei Temperaturen zwischen Raumtemperatur und 50 °C, durchgeführt.

Die anschließende Hydrolyse wird zweckmäßigerweise entweder in Gegenwart einer Säure wie Salzsäure, Schwefelsäure, Phosphorsäure, Trichloressigsäure oder Trifluoressigsäure in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 °C und 120 °C, z.B. bei Temperaturen zwischen Raumtemperatur und der Siedetemperatur des Reaktionsgemisches, durchgeführt.

Bei der Umsetzung erhält man vorzugsweise ein Gemisch der 1- und 3-Isomeren, welches gewünschtenfalls anschließend, vorzugsweise chromatographisch unter Verwendung eines Trägers wie Kieselgel oder Aluminiumoxid, in das entsprechende 1- und 3-Isomere aufgetrennt wird.

b) Zur Herstellung einer Verbindung der allgemeinen Formel I, in der $R_b$ eine Carboxygruppe darstellt: Überführung einer Verbindung der allgemeinen Formel

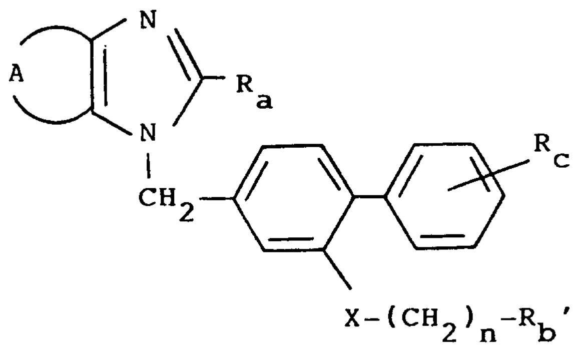

,(IV)

in der
n, X, A, $R_a$ und $R_c$ wie eingangs definiert sind und $R_b$' eine mittels Hydrolyse, Thermolyse oder Hydrogenolyse in eine Carboxygruppe überführbare Gruppe darstellt, in eine entsprechende Carboxyverbindung.

Beispielsweise können funktionelle Derivate der Carboxygruppe wie deren unsubstituierte oder substituierte Amide, Ester, Thiolester, Orthoester, Iminoäther, Amidine oder Anhydride, die Nitrilgruppe oder die Tetrazolylgruppe mittels Hydrolyse in eine Carboxygruppe, Ester mit tertiären Alkoholen, z.B. der tert. Butylester, mittels Thermolyse in eine Carboxygruppe und Ester mit Aralkanolen, z.B. der Benzylester, mittels Hydrogenolyse in eine Carboxygruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 °C und 120 °C, z.B. bei Temperaturen zwischen Raumtemperatur und der Siedetemperatur des Reaktionsgemisches, durchgeführt. Bei der Hydrolyse in Gegenwart einer organischen Säuren wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet $R_b$' in einer Verbindung der allgemeinen Formel IV eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50 °C in die Carboxygruppe übergeführt werden.

Bedeutet $R_b$' in einer Verbindung der allgemeinen Formel IV beispielsweise die tert. Butyloxycarbonylgruppe, so kann die tert. Butylgruppe auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 °C und 100 °C, abgespalten werden.

Bedeutet $R_b$' in einer Verbindung der allgemeinen Formel IV beispielsweise die Benzyloxycarbonylgruppe, so kann die Benzylgruppe auch hydrogenolytisch in Gegenwart eines Hydrierungskatalysators

wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50 ° C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, eine Benzyloxygruppe zur Hydroxygruppe, eine Vinylidengruppe zur entsprechenden Alkylidengruppe oder eine Zimtsäuregruppe zur entsprechenden Phenyl-propionsäuregruppe, mitreduziert oder durch Wasserstoffatome, z.B. ein Halogenatom durch ein Wasserstoffatom, ersetzt werden.

c) Zur Herstellung einer Verbindung der allgemeinen Formel I, in der $R_b$ eine 1H-Tetrazolylgruppe darstellt:

Abspaltung eines Schutzrestes von einer Verbindung der allgemeinen Formel

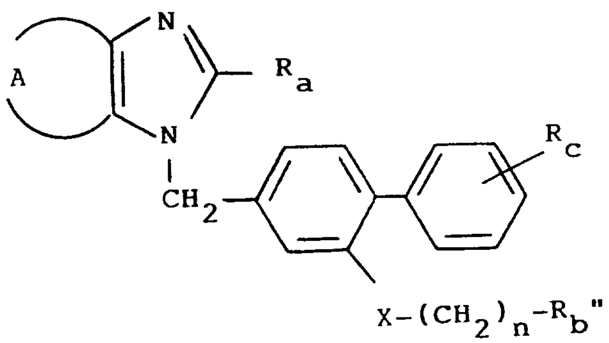

,(V)

in der

n, X, A, $R_a$ und $R_c$ wie eingangs definiert sind und $R_b''$ eine in 1- oder 2-Stellung durch einen Schutzrest geschützte 1H-Tetrazolylgruppe darstellt.

Als Schutzrest kommt beispielsweise die β-Cyanoethyl-, Triphenylmethyl-, Tributylzinn- oder Triphenylzinngruppe in Betracht.

Die Abspaltung eines verwendeten Schutzrestes erfolgt vorzugsweise in Gegenwart eines Halogenwasserstoffes, vorzugsweise in Gegenwart von Chlorwasserstoff, in Gegenwart einer Base wie Natriumhydroxid oder alkoholischem Ammoniak in einem geeigneten Lösungsmittel wie Methylenchlorid, Methanol, Methanol/Ammoniak, Ethanol oder Isopropanol bei Temperaturen zwischen 0 und 100 ° C, vorzugsweise jedoch bei Raumtemperatur, oder auch, falls die Umsetzung in Gegenwart von alkoholischem Ammoniak durchgeführt wird, bei erhöhten Temperaturen, z.B. bei Temperaturen zwischen 100 und 150 ° C, vorzugsweise bei Temperaturen zwischen 120 und 140 ° C.

d) Zur Herstellung einer Verbindung der allgemeinen Formel I, in der $R_b$ eine 1H-Tetrazolylgruppe darstellt:

Umsetzung einer Verbindung der allgemeinen Formel

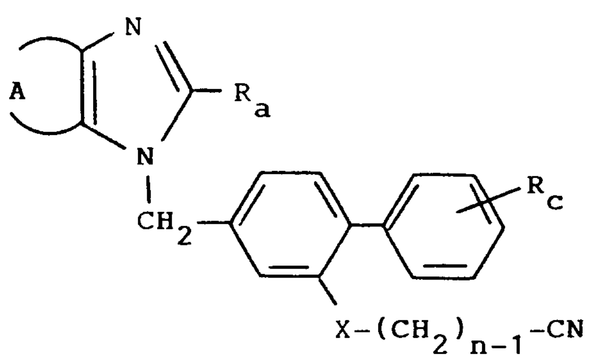

,(VI)

in der

n, X, A, $R_a$ und $R_c$ wie eingangs definiert sind, mit Stickstoffwasserstoffsäure oder deren Salzen.

Die Umsetzung wird vorzugsweise in einem Lösungsmittel wie Benzol, Toluol oder Dimethylformamid bei Temperaturen zwischen 80 und 150 ° C, vorzugsweise bei 125 ° C, durchgeführt. Hierbei wird zweckmäßigerweise entweder die Stickstoffwasserstoffsäure während der Umsetzung aus einem Alkaliazid, z.B. aus Natriumazid, in Gegenwart einer schwachen Säure wie Ammoniumchlorid freigesetzt oder das im Reaktionsgemisch bei der Umsetzung mit einem Salz der Stickstoffwassersäure, vorzugsweise mit Aluminiumazid oder Tributylzinnazid, welche außerdem zweckmäßigerweise im Reaktionsgemisch durch Umsetzung von Aluminiumchlorid oder Tributylzinnchlorid mit einem Alkaliazid wie Natriumazid hergestellt werden, erhaltene Tetrazolidsalz anschließend durch Ansäuern mit einer verdünnten Säure wie 2N Salzsäure oder 2N Schwefelsäure freigesetzt.

e) Zur Herstellung einer Verbindung der allgemeinen Formel I, in der n die Zahl 1, X eine Bindung und $R_b$ eine Bis(hydroxycarbonyl)methyl- oder Bis(alkoxycarbonyl)methyl-Gruppe darstellen:

Umsetzung einer Verbindung der allgemeinen Formel

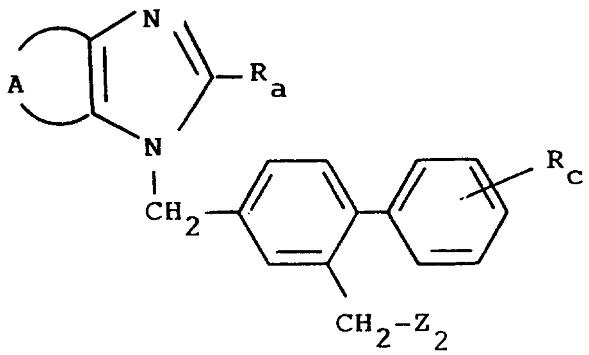

,(VII)

in der

n, A, $R_a$ und $R_c$ wie eingangs definiert sind und $Z_2$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe, bedeuten, mit einer Verbindung der allgemeinen Formel

$CH_2(COOR_6)_2$ ,(VIII)

in der

$R_6$ jeweils eine Alkylgruppe mit 1 bis 4 Kohlenstoffatomen darstellt, erforderlichenfalls anschließende Hydrolyse und/oder Decarboxylierung.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid oder Benzol gegebenenfalls in Gegenwart eines säurebindenden Mittels, wie Natriumcarbonat, Kaliumcarbonat, Natriumhydroxid, Kalium-tert.butylat, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und 50°C, durchgeführt.

Die anschließende Hydrolyse wird zweckmäßigerweise entweder in Gegenwart einer Säure wie Salzsäure, Schwefelsäure, Phosphorsäure, Trichloressigsäure oder Trifluoressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10°C und 120°C, z.B. bei Temperaturen zwischen Raumtemperatur und der Siedetemperatur des Reaktionsgemisches, durchgeführt.

Die anschließende Decarboxylierung wird zweckmäßigerweise in Gegenwart einer Säure wie Salzsäure, Schwefelsäure, Phosphorsäure, Essigsäure, Trichloressigsäure oder Trifluoressigsäure in einem geeigneten Lösungsmittel wie Wasser, Wasser/Methanol, Äthanol, Wasser/Äthanol, Wasser/Isopropanol oder Wasser/Dioxan bei Temperaturen zwischen 50°C und 120°C, z.B. bei Temperaturen zwischen 60°C und der Siedetemperatur des Reaktionsgemisches, durchgeführt.

f) Zur Herstellung einer Verbindung der allgemeinen Formel I, in der $R_2$ eine $R_5$-$NR_4$-$CONR_3$-Gruppe darstellt:

Umsetzung einer Verbindung der allgemeinen Formel

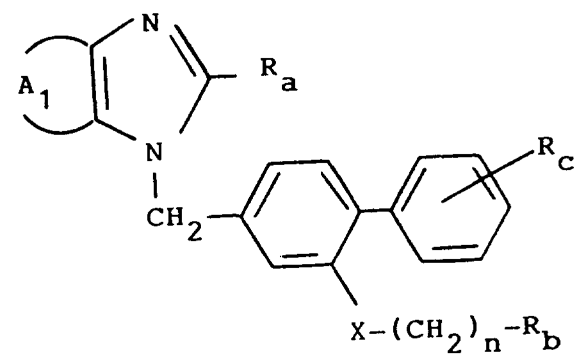

,(IX)

mit einer Verbindung der allgemeinen Formel

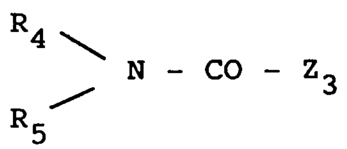

,(X)

in denen

$R_a$, $R_b$, $R_c$, $R_4$, $R_5$, X und n wie eingangs definiert sind, $A_1$ eine 1,4-Butadienylengruppe, die durch $R_1$ und durch die $R_3$NH-Gruppe substituiert ist, wobei $R_1$ und $R_3$ wie eingangs definiert sind, und

$Z_3$ eine nukleophile Austrittsgruppe wie ein Chlor- oder Bromatom oder auch

$Z_3$ und $R_5$ zusammen eine Stickstoff-Kohlenstoff-Bindung darstellen.

Die Umsetzung wird zweckmäßigerweise in einem Lösungsmittel oder Lösungsmittelgemisch wie Dichlormethan, Chloroform, Diethylether, Tetrahydrofuran, Dioxan, Dimethylformamid, Dimethylsulfoxid, Pyridin, Benzol oder Toluol bei Temperaturen zwischen 0 und 150°C, vorzugsweise jedoch bei Temperaturen zwischen 50 und 120°C durchgeführt.

g) Zur Herstellung einer Verbindung der allgemeinen Formel I, in der $R_2$ eine Alkanoylaminogruppe mit 2 bis 5 Kohlenstoffatomen im Alkanoylteil oder eine Benzolsulfonylaminogruppe, welche beide am Stickstoffatom durch eine Alkylgruppe mit 1 bis 3 Kohlenstoffatomen substituiert sein können, eine Phthalimino- oder Homophthaliminogruppe, wobei eine Carbonylgruppe in einer Phthaliminogruppe durch eine Methylengruppe ersetzt sein kann, eine 5-, 6- oder 7-gliedrige Alkyleniminogruppe, in welcher eine Methylengruppe durch eine Carbonyl- oder Sulfonylgruppe ersetzt ist, eine Glutarsäureiminogruppe, in der die n-Propylengruppe durch ein oder zwei Alkylgruppen mit jeweils 1 bis 3 Kohlenstoffatomen oder durch eine Tetramethylen- oder Pentamethylengruppe substituiert sein kann, oder eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können, darstellt:

Umsetzung einer Verbindung der allgemeinen Formel

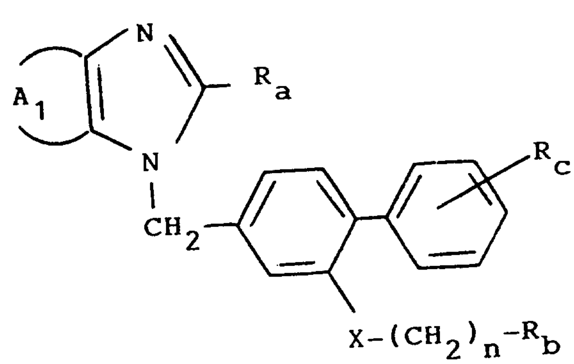

,(IX)

mit einer Verbindung der allgemeinen Formel

$Z_4$ - U - $R_7$ ,(XI)

in denen

$R_a$, $R_b$, $R_c$, $R_4$, $R_5$, X und n wie eingangs definiert sind, $A_1$ eine 1,4-Butadienylengruppe, die durch $R_1$ und durch die $R_3$NH-Gruppe substituiert ist, wobei $R_1$ und $R_3$ wie eingangs definiert sind,

$Z_4$ eine Hydroxygruppe oder eine nukleophile Austrittsgruppe wie ein Halogenatom, z. B. ein Chlor- oder Bromatom,

U eine Carbonyl- oder Sulfonylgruppe und

$R_7$ eine Alkylgruppe mit 1 bis 4 Kohlenstoffatomen, eine Phenyl-, o-Hydroxycarbonylphenyl-, o-Hydroxycarbonylphenylmethyl- oder o-Hydroxycarbonylmethylphenylgruppe, eine gegebenenfalls durch eine oder zwei Alkylgruppen mit jeweils 1 bis 3 Kohlenstoffatomen oder durch eine Tetramethylen- oder Pentamethylengruppe substituierte 3-Hydroxycarbonylpropylengruppe oder eine gegebenenfalls durch eine Alkylgruppe mit 1 bis 3 Kohlenstoffatomen oder durch eine Phenylgruppe mono- oder disbustituierte 2-Hydroxycarbonylethenylengruppe, in der die Substituenten gleich oder verschieden sein können, oder

$R_3$ und $R_7$ zusammen eine n-Propylen-, n-Butylen- oder n-Hexylengruppe bedeuten, oder, falls $Z_4$ eine Hydroxygruppe darstellt, mit deren reaktionsfähigen Derivaten wie deren Säurehalogeniden, Säureanhydriden oder Säureestern.

Als reaktionsfähige Derivate einer Verbindung der Formel XI kommen beispielsweise deren Ester wie der Methyl-, Ethyl- oder Benzylester, deren Thioester wie der Methylthio- oder Ethylthioester, deren Halogenide wie das Säurechlorid, deren Anhydride oder Imidazolide und deren Orthoester in Betracht.

Die Umsetzung wird zweckmäßigerweise in einem Lösungsmittel oder Lösungsmittelgemisch wie Wasser, Methylenchlorid, Chloroform, Äther, Tetrahydrofuran, Dioxan oder Dimethylformamid oder in einem Überschuß des Acylierungsmittels als Lösungsmittel mit einer entsprechenden Carbonsäure in Gegenwart eines die Säure aktivierenden oder wasserentziehenden Mittels wie Thionylchlorid, mit deren Anhydriden wie Essigsäureanhydrid, mit deren Estern wie Essigsäureäthylester, mit deren Halogeniden wie Acetylchlorid oder Methansulfonylchlorid gegebenenfalls in Gegenwart einer anorganischen oder tertiären organischen Base, wie Natriumhydroxid, Kaliumcarbonat, Triäthylamin oder Pyridin, wobei die beiden letzteren gleichzeitig auch als Lösungsmittel dienen können, bei Temperaturen zwischen -25 und 100°C, vorzugsweise jedoch bei Temperaturen zwischen -10 und 80°C, durchgeführt.

Erhält man erfindungsgemäß eine Verbindung der allgemeinen Formel I, in der $R_2$ oder $R_b$ oder $R_2$ und $R_b$ jeweils eine Carboxygruppe darstellen, so kann diese mittels Veresterung in eine entsprechende Verbindung der allgemeinen Formel I, in der $R_2$ oder $R_b$ oder $R_2$ und $R_b$ eine in vivo in eine Carboxygruppe überführbare Gruppe darstellen, übergeführt werden.

Die Überführung einer Carboxylgruppe in eine Gruppe, die in vivo metabolisch in eine Carboxygruppe umgewandelt wird, erfolgt zweckmäßigerweise durch Veresterung mit einem entsprechenden Alkohol oder mit einem entsprechenden reaktionsfähigen Acylderivat zweckmäßigerweise in einem Lösungsmittel oder Lösungsmittelgemisch wie Wasser, Methylenchlorid, Chloroform, Äther, Tetrahydrofuran, Dioxan oder Dimethylformamid oder in einem Überschuß des Acylierungsmittels als Lösungsmittel gegebenenfalls in Gegenwart eines die Säure aktivierenden oder wasserentziehenden Mittels wie Thionylchlorid, mit deren Anhydriden, Estern oder Halogeniden gegebenenfalls in Gegenwart einer anorganischen oder tertiären organischen Base, wie Natriumhydroxid, Kaliumcarbonat, Triäthylamin oder Pyridin, wobei die beiden letzteren gleichzeitig auch als Lösungsmittel dienen können, bei Temperaturen zwischen -25 und 100°C, vorzugsweise jedoch bei Temperaturen zwischen -10 und 80°C, durchgeführt.

Bei den vorstehend beschriebenen Umsetzungen können gegebenenfalls vorhandene reaktive Gruppen wie Hydroxy-, Amino- oder Alkylaminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Benzyl- oder Tetrahydropyranylgruppe und als Schutzrest für eine Amino-, Alkylamino- oder Iminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

Ein so erhaltenes Isomerengemisch einer Verbindung der allgemeinen Formel I kann gewünschtenfalls vorzugsweise chromatographisch unter Verwendung eines Trägers wie Kieselgel oder Aluminiumoxid getrennt werden.

Desweiteren können die erhaltenen Verbindungen der allgemeinen Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel I, falls diese eine Carboxy- oder 1H-Tetrazolylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis XI sind teilweise literaturbekannt oder man erhält diese nach literaturbekannten Verfahren.

So erhält man beispielsweise eine Verbindung der allgemeinen Formel II durch Acylierung einer entsprechenden o-Amino-nitroverbindung, anschließende Reduktion der Nitrogruppe und anschließende Cyclisierung oder durch Umsetzung einer entsprechenden o-Diaminoverbindung mit einem entsprechenden Tetraalkyl-orthokohlensäureester.

Eine Verbindung der allgemeinen Formel II, in der $R_a$ eine Amino- oder Alkylaminogruppe darstellt, erhält man durch Cyclisierung einer entsprechenden o-Diaminoverbindung mit einem Kohlensäurediester, anschließende Halogenierung der so erhaltenen 2-Hydroxyverbindung und Umsetzung mit einem entsprechenden Amin.

Die als Ausgangsstoffe verwendeten Verbindungen der allgemeinen Formeln IV, V, VI, VII und IX erhält man durch Cyclisierung eines entsprechenden o-Phenylendiamins oder durch Reduktion einer entsprechenden o-Amino-nitroverbindung, anschließender Reduktion der Nitrogruppe und Cyclisierung einer so erhaltenen o-Diaminophenylverbindung oder durch NH-Alkylierung eines entsprechenden 1H-Benzimidazols, wobei das so erhaltene Isomerengemisch anschließend mittels üblicher Methoden, z.B. mittels Chromatographie, aufgetrennt werden kann.

Die neuen Verbindungen der allgemeinen Formel I und deren physiologisch verträgliche Salze weisen wertvolle pharmakologische Eigenschaften auf. Sie stellen Angiotensin-Antagonisten, insbesondere Angiotensin-II-Antagonisten, dar.

Beispielsweise wurden die Verbindungen

A = 2-n-Propyl-4-methyl-1-[3-(tetrazol-5-yl-methyl)-4-phenyl-benzyl]-benzimidazol,

B = 2-n-Propyl-4-methyl-1-[3-(2-hydroxycarbonyl-ethyl)-4-phenyl-benzyl]-benzimidazol,

C = 2-n-Propyl-4-methyl-6-(1-methyl-benzimidazol-2-yl)-1-[3-(2-hydroxycarbonyl-ethyl)-4-phenyl-benzyl]-benzimidazol,

D = 2-Ethyl-5,7-dimethyl-3-[3-(tetrazol-5-yl-methyl)-4-phenyl-benzyl]-imidazo[4,5-b]pyridin und

E = 2-n-Propyl-4-methyl-6-(1-isopropyl-imidazol-4-yl)-1-[3-(2-tetrazol-5-yl-ethyl)-4-phenyl-benzyl]-benzimidazol

auf ihre biologischen Wirkungen wie folgt untersucht:

Methodenbeschreibung Angiotensin II-Rezeptorbindung

Das Gewebe (Rattenlunge) wird in Tris-Puffer (50 mMol Tris, 150 mMol NaCl, 5 mMol EDTA, pH 7.40) homogenisiert und zweimal je 20 Min. bei 20.000 x g zentrifugiert. Das endgültige Pellet wird in Inkubations-Puffer (50 mMol Tris, 5 mMol $MgCl_2$, 0,2 % BSA, pH 7.40) 1:75, bezogen auf das Feuchtgewicht des Gewebes, resuspendiert. Je 0,1 ml Homogenat wird für 60 Min. bei 37°C mit 50 pM [$125^I$]-Angiotensin II (NEN, Dreieich, FRG) und steigenden Konzentrationen der Testsubstanz in einem Gesamtvolumen von 0,25 ml inkubiert. Die Inkubation wird durch rasche Filtration durch Glasfiber-Filtermatten beendet. Die Filter werden je 4 ml eiskaltem Puffer (25 mMol Tris, 2.5 mMol $MgCl_2$, 0,1 % BSA, pH 7,40) gewaschen. Die gebundene Radioaktivität wird in einem Gamma-Counter ermittelt. Aus der Dosis-Wirkungskurve wird der entsprechende $IC_{50}$-Wert ermittelt.

Die Substanzen A bis E zeigen in dem beschriebenen Test folgende $IC_{50}$-Werte:

| Substanz | $IC_{50}$ [nM] |
|---|---|
| A | 370 |
| B | 270 |
| C | 18 |
| D | 73 |
| E | 6,9 |

Desweiteren konnten bei der Applikation der vorstehenden Verbindungen bis zu einer Dosis von 30 mg/kg i.v. keine toxischen Nebenwirkungen, z.B. keine negativ inotrope Wirkung und keine Herzrhythmusstörungen, beobachtet werden. Die Verbindungen sind demnach gut verträglich.

Aufgrund ihrer pharmakologischen Eigenschaften eignen sich die neuen Verbindungen und deren physiologisch verträgliche Salze zur Behandlung der Hypertonie und Herzinsuffizienz, ferner zur Behandlung ischämischer peripherer Durchblutungs-störungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen.

Weiterhin eignen sich die neuen Verbindungen und deren physiologisch verträgliche Salze zur Behandlung pulmonaler Erkrankungen, z.B. von Lungenödemen und der chronischen Bronchitis, zur Prävention von arterieller Re-Stenosis nach Angioplastie, von Verdickungen der Gefäßwand nach Gefäßoperationen, der Arteriosklerose und der diabetischen Angiopathie. Auf Grund der Beeinflußung der Acetylcholin- und Dopamin-Freisetzung durch Angiotensin im Gehirn eignen sich die neuen Angiotensin-Antagonisten auch zur Behebung zentral-nervöser Störungen, z.B. von Depressionen, der Alzheimer'schen Krankheit, des Parkinson-Syndroms, der Bulimie, sowie von Störungen kognitiver Funktionen.

Die zur Erzielung einer entsprechenden Wirkung am Erwachsenen erforderliche Dosierung beträgt zweckmäßigerweise bei intravenöser Gabe 20 bis 100 mg, vorzugsweise 30 bis 70 mg, und bei oraler Gabe 50 bis 200 mg, vorzugsweise 75 bis 150 mg, jeweils 1 bis 3 x täglich. Hierzu lassen sich die erfindungsgemäß hergestellten Verbindungen der allgemeinen Formel I, gegebenenfalls in Kombination mit anderen Wirksubstanzen wie z.B. Blutdrucksenker, Diuretika und/oder Kalzium-Antagonisten, zusammen mit einem oder mehreren inerten üblichen Trägerstoffen und/oder Verdünnungsmitteln, z.B. mit Maisstärke, Milchzukker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Für die oben erwähnten Kombinationen kommen somit als weitere Wirksubstanzen beispielsweise Bendroflumethiazid, Chlorthiazid, Hydrochlorthiazid, Spironolacton, Benzthiazid, Cyclothiazid, Ethacrinsäure, Furosemid, Metoprolol, Prazosin, Atenolol, Propranolol, (Di)hydralazin-hydrochlorid, Diltiazem, Felodipin, Nicardipin, Nifedipin, Nisoldipin und Nitrendipin in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 15 bis 200 mg Hydrochlorthiazid, 125 bis 2000 mg Chlorthiazid, 15 bis 200 mg Ethacrinsäure, 5 bis 80 mg Furosemid, 20 bis 480 mg Propranolol, 5 bis 60 mg Felodipin, 5 bis 60 mg Nifedipin oder 5 bis 60 mg Nitrendipin.

Die nachfolgenden Beispiele sollen die Erfindung näher erläutern:

## Beispiel 1

### 2-n-Propyl-4-methyl-1-(3-hydroxycarbonyl-4-phenyl-benzyl)-benzimidazol

a) 3-Methoxycarbonyl-4-phenyl-benzylbromid

1,0 g (4,4 mMol) 3-Methoxycarbonyl-4-phenyl-toluol, 890 mg (5,0 mMol) N-Bromsuccinimid und ca. 30 mg Azo-bis-isobutyronitril werden in 30 ml Tetrachlorkohlenstoff 30 Minuten lang unter UV-Bestrahlung zum Rückfluß erhitzt. Anschließend wird vom entstandenen Succinimid abfiltriert, das Filtrat zweimal mit je 50 ml Wasser gewaschen, getrocknet und bis zur Trockne eingeengt. Das so erhaltene Produkt, in dem noch ca. 10 % Ausgangsmaterial enthalten waren, wird ohne weitere Reinigung weiter umgesetzt.
Ausbeute: 1,3 g (100 % der Theorie),
$R_f$-Wert: 0,34 (Kieselgel; Petrolether/Essigester = 9:1)

b) 2-n-Propyl-4-methyl-1-(3-methoxycarbonyl-4-phenyl-benzyl)-benzimidazol

Eine Lösung von 10,45 g (60 mMol) 2-n-Propyl-4-methyl-benzimidazol, 8,1 g (72 mMol) Kalium-tert.butylat und 22,0 g (72 mMol) 3-Methoxycarbonyl-4-phenyl-benzylbromid in 300 ml N,N-Dimethylformamid wird ca. 2 Stunden lang bei Raumtemperatur gerührt und anschließend in ca. 600 ml Wasser eingerührt. Das so erhaltene Gemisch wird viermal mit je ca. 60 ml Essigester extrahiert, die vereinigten organischen Extrakte mit ca. 50 ml Wasser gewaschen, getrocknet und eingeengt. Das so erhaltene Rohprodukt wird durch Säulenchromatographie (1 kg Kieselgel; Laufmittel: Petrolether/Essigester = 2:1) gereinigt.
Ausbeute: 16,8 g (70 % der Theorie),
Öl, $R_f$-Wert: 0,30 (Kieselgel; Petrolether/Essigester = 2:1)

c) 2-n-Propyl-4-methyl-1-(3-hydroxycarbonyl-4-phenyl-benzyl)-benzimidazol

1,00 g (2,5 mMol) 2-n-Propyl-4-methyl-1-(3-methoxycarbonyl-4-phenyl-benzyl)-benzimidazol wird 1,5 Stunden lang in einem Gemisch aus 10 ml 2N Natronlauge und 5 ml Methanol zum Rückfluß erhitzt. Anschließend destilliert man das Methanol ab, fügt ca. 10 ml Wasser hinzu und säuert mit Eisessig an. Das dabei ausgefallene Produkt wird abgesaugt, mit ca. 5 ml Wasser gewaschen und bei 60 °C getrocknet.
Ausbeute: 580 mg (60 % der Theorie),
Schmelzpunkt: 258-260 °C

| $C_{25}H_{24}N_2O_2$ (384,49) | | | | | | |
|---|---|---|---|---|---|---|
| Ber.:<br>Gef.: | C | 78,09<br>77.92 | H | 6,29<br>6,44 | N | 7,29<br>7,33 |

$R_f$-Wert: 0,54 (Kieselgel; Methylenchlorid/Ethanol = 9:1)

Beispiel 2

2-n-Propyl-4-methyl-1-[3-(tetrazol-5-yl-methyl)-4-phenyl-benzyl]-benzimidazol

a) 2-n-Propyl-4-methyl-1-(3-chlormethyl-4-phenyl-benzyl)-benzimidazol

Eine Lösung von 3,2 g (8,6 mMol) 2-n-Propyl-4-methyl-1-(3-hydroxymethyl-4-phenyl-benzyl)-benzimidazol in 5 ml Thionylchlorid wird 10 Minuten lang auf 100 °C erhitzt. Anschließend destilliert man das überschüssige Thionylchlorid ab, verrührt den erhaltenen Rückstand mit ca. 20 g Eis und neutralisiert die so erhaltene Lösung mit 5%iger Natriumhydrogencarbonat-Lösung. Anschließend extrahiert man dreimal mit je ca. 15 ml Methylenchlorid, trocknet die vereinigten organischen Extrakte, filtriert über Aktivkohle und engt ein.
Ausbeute: 3,3 g (100 % der Theorie),
Öl, $R_f$-Wert: 0,63 (Kieselgel; Petrolether/Essigester = 1:1)

b) 2-n-Propyl-4-methyl-1-(3-cyanmethyl-4-phenyl-benzyl)-benzimidazol

Zu einer Lösung von 3,3 g (8,6 mMol) 2-n-Propyl-4-methyl-1-(3-chlormethyl-4-phenyl-benzyl)-benzimidazol in 10 ml Dimethylsulfoxid werden 640 mg (13 mMol) Natriumcyanid gegeben und das Gemisch zwei Stunden lang bei 80 °C gerührt, dann in ca. 40 ml 5%ige Natriumchlorid-Lösung eingerührt und viermal mit je ca. 20 ml Methylenchlorid extrahiert. Die vereinigten organischen Extrakte werden mit 30 ml einer Eisen-(II)-Sulfat-Lösung gewaschen, getrocknet und eingeengt. Das so erhaltene Rohprodukt wird durch Säulenchromatographie (150 g Kieselgel; Laufmittel: Petrolether/Essigester = 2:1) gereinigt.
Ausbeute: 2,2 g (68 % der Theorie),
Öl, $R_f$-Wert: 0,50 (Kieselgel; Petrolether/Essigester = 1:1)

c) 2-n-Propyl-4-methyl-1-[3-(tetrazol-5-yl-methyl)-4-phenyl-benzyl]-benzimidazol

Eine Lösung von 1,2 g (3,2 mMol) 2-n-Propyl-4-methyl-1-(3-cyanomethyl-4-phenyl-benzyl)-benzimidazol, 3,4 g (64 mMol) Ammoniumchlorid und 4,2 g (64 mMol) Natriumazid in 25 ml Dimethylformamid wird

drei Stunden lang bei 140 °C gerührt. Anschließend wird in ca. 60 ml einer 5%igen Natriumchlorid-Lösung eingerührt, das ausgefallene Rohprodukt abgesaugt und durch Säulenchromatographie (100 g Kieselgel; Laufmittel: Methylenchlorid/Ethanol = 20:1) gereinigt.
Ausbeute: 1,0 g (75 % der Theorie),
Schmelzpunkt: 183-185 °C

| $C_{26}H_{26}N_6$ (422,55) | | | | | | |
|---|---|---|---|---|---|---|
| Ber.:<br>Gef.: | C | 73,90<br>73,81 | H | 6,20<br>6,44 | N | 19,89<br>19,78 |

$R_f$-Wert: 0,53 (Kieselgel; Methylenchlorid/Ethanol = 9:1) Massenspektrum: m/e = 422

Beispiel 3

2-n-Propyl-4-methyl-1-[3-(2-hydroxycarbonyl-ethyl)-4-phenyl-benzyl]-benzimidazol

a) 2-n-Propyl-4-methyl-1-[3-(2,2-bis-ethoxycarbonyl-ethyl)-4-phenyl-benzyl]-benzimidazol

Zu einer Lösung von 6,24 g (39 mMol) Malonsäure-diethylester und 4,4 g (39 mMol) Kalium-tert.butylat in 75 ml Dimethylsulfoxid wird bei Raumtemperatur eine Lösung von 12,6 g (32,4 mMol) 2-n-Propyl-4-methyl-1-(3-chlormethyl-4-phenyl-benzyl)-benzimidazol in 25 ml Dimethylsulfoxid zugetropft. Nach Rühren über Nacht wird das Reaktionsgemisch in ca. 400 ml einer 5%igen Natriumchloridlösung eingerührt, das Gemisch dann dreimal mit je ca. 80 ml Essigester extrahiert, die organischen Extrakte mit ca. 100 ml Wasser gewaschen, eingeengt und durch Säulenchromatographie (500 g Kieselgel; Laufmittel: Petrolether/Essigester = 1:1) gereinigt.
Ausbeute: 7,0 (42 % der Theorie),
Öl, $R_f$-Wert: 0,51 (Kieselgel; Petrolether/Essigester = 1:1) Öl, $R_f$-Wert: 0,83 (Kieselgel; Methylenchlorid/Ethanol = 4:1)

b) 2-n-Propyl-4-methyl-1-[3-(2,2-bis-hydroxycarbonyl-ethyl)-4-phenyl-benzyl]-benzimidazol

Ein Gemisch aus 7,0 g (13,6 mMol) 2-n-Propyl-4-methyl-1-[3-(2,2-bis-ethoxycarbonyl-ethyl)-4-phenyl-benzyl]-benzimidazol, 20 ml Ethanol und 25 ml 2N Natronlauge wird 90 Minuten lang unter Rückfluß erhitzt. Anschließend wird eingedampft, der Rückstand in ca. 60 ml Wasseer gelöst und diese Lösung einmal mit ca. 30 ml Diethylether gewaschen. Die wäßrige Phase wird dann mit Eisessig angesäuert, das auskristallisierte Produkt abgesaugt, mit ca. 40 ml Wasser gewaschen und getrocknet.
Ausbeute: 5,1 g (82 % der Theorie),
$R_f$-Wert: 0,50 (Kieselgel; Methylenchlorid/Ethanol = 4:1)

c) 2-n-Propyl-4-methyl-1-[3-(2-hydroxycarbonyl-ethyl)-4-phenyl-benzyl]-benzimidazol

5,1 g (11 mMol) 2-n-Propyl-4-methyl-1-[3-(2,2-bis-hydroxycarbonyl-ethyl)-4-phenyl-benzyl]-benzimidazol werden 30 Minuten lang auf 140 °C erhitzt. Das nach dem Abkühlen erhaltene feste Produkt wird mit Diethylether verrieben, abgesaugt und getrocknet.
Ausbeute: 4,1 g (91 % der Theorie),
Schmelzpunkt: 201-203 °C

| $C_{27}H_{28}N_2O_2$ (412,54) | | | | | | |
|---|---|---|---|---|---|---|
| Ber.:<br>Gef.: | C | 78,62<br>78,57 | H | 6,84<br>6,90 | N | 6,79<br>6,79 |

Massenspektrum: m/e = 412

Beispiel 4

2-n-Propyl-4-methyl-1-[3-(2-tetrazol-5-yl-ethyl)-4-phenyl-benzyl]-benzimidazol

a) 2-n-Propyl-4-methyl-1-[3-(2-aminocarbonyl-ethyl)-4-phenyl-benzyl]-benzimidazol

Eine Mischung aus 3,0 g (7,3 mMol) 2-n-Propyl-4-methyl-1-[3-(2-hydroxycarbonyl-ethyl)-4-phenyl-benzyl]-benzimidazol, 4,5 ml Thionylchlorid, 60 ml Methylenchlorid und 3 Tropfen Dimethylformamid wird eine Stunde lang unter Rückfluß erhitzt und dann bis zur Trockne eingeengt. Der erhaltene Rückstand wird in 10 ml Tetrahydrofuran gelöst und unter Rühren in 30 ml konzentriertem Ammoniak eingetropft. Nach beendeter Zugabe wird mit ca. 30 ml Wasser verdünnt, anschließend das Tetrahydrofuran abdestilliert, das ausgefallene Produkt abgesaugt, mit Wasser gewaschen und bei 60 °C getrocknet.
Ausbeute: 2,9 (97 % der Theorie),
$R_f$-Wert: 0,41 (Kieselgel; Methylenchlorid/Ethanol = 19:1)

b) 2-n-Propyl-4-methyl-1-[3-(2-cyanoethyl)-4-phenyl-benzyl]-benzimidazol

Eine Lösung von 2,9 g (7,0 mMol) 2-n-Propyl-4-methyl-1-[3-(2-aminocarbonyl-ethyl)-4-phenyl-benzyl]-benzimidazol in 40 ml Phosphoroxychlorid wird 45 Minuten lang zum Rückfluß erhitzt. Anschließend wird das überschüssige Phosphoroxychlorid abdestilliert, der Rückstand mit ca. 40 ml Eiswasser zersetzt und die Mischung dann unter Kühlung mit konzentriertem Ammoniak alkalisch gestellt. Die wäßrige Phase wird vom ausgefallenen Rohprodukt abdekantiert, der Rückstand in ca. 60 ml Methylenchlorid gelöst, über Aktivkohle filtriert und zur Trockne eingeengt.
Ausbeute: 2,7 g (98 % der Theorie),
$R_f$-Wert: 0,46 (Kieselgel; Methylenchlorid/Ethanol = 19:1)

c) 2-n-Propyl-4-methyl-1-[3-(2-tetrazol-5-yl-ethyl)-4-phenyl-benzyl]-benzimidazol

Eine Lösung von 2,7 g (6,86 mMol) 2-n-Propyl-4-methyl-1-[3-(2-cyanoethyl)-4-phenyl-benzyl]-benzimidazol, 7,4 g (137 mMol) Ammoniumchlorid und 9,0 g (137 mMol) Natriumazid in 80 ml Dimethylformamid wird vier Stunden lang auf 140 °C erhitzt, dann in ca. 200 ml 5%ige Natriumchloridlösung eingerührt und das Gemisch viermal mit je ca. 40 ml Methylenchlorid extrahiert. Die vereinigten organischen Extrakte werden mit ca. 100 ml Wasser gewaschen, getrocknet, eingeengt und das so erhaltene Rohprodukt durch Säulenchromatographie (130 g Kieselgel; Laufmittel = Methylenchlorid + 5 bis 8 % Ethanol) gereinigt.
Ausbeute: 1,0 g (33 % der Theorie),
Schmelzpunkt: ab 78 °C sintern

| $C_{27}H_{28}N_6$ (436,57) | | | | | | |
|---|---|---|---|---|---|---|
| Ber.:<br>Gef.: | C | 74,28<br>73,94 | H | 6,46<br>6,61 | N | 19,25<br>18,98 |

Massenspektrum: m/e = 436

Beispiel 5

2-n-Propyl-4-methyl-6-(1-methyl-benzimidazol-2-yl)-1-[3-(2-hydroxycarbonyl-ethyl)-4-phenyl-benzyl]-benzimidazol

Hergestellt analog Beispiel 3 aus 2-n-Propyl-4-methyl-6-(1-methyl-benzimidazol-2-yl)-1-[3-(2,2-bis-hydroxycarbonyl-ethyl)-4-phenyl-benzyl]-benzimidazol.
Ausbeute: 80 % der Theorie,
Schmelzpunkt: ab 114 °C sintern

| $C_{35}H_{34}N_4O_2$ (542,69) | | | | | | |
|---|---|---|---|---|---|---|
| Ber.:<br>Gef.: | C | 77,46<br>77,03 | H | 6,31<br>6,41 | N | 10,32<br>10,09 |

$R_f$-Wert: 0,86 (Kieselgel; Methylenchlorid/Ethanol = 9:1) Massenspektrum: $(M+H)^+$ = 543

Beispiel 6

2-n-Propyl-4-methyl-6-(1-isopropyl-imidazol-4-yl)-1-[3-(tetrazol-5-yl-methyl)-4-phenyl-benzyl]-benzimidazol

Hergestellt analog Beispiel 2 aus 2-n-Propyl-4-methyl-6-(1-isopropyl-imidazol-4-yl)-1-(3-cyanomethyl-4-phenyl-benzyl)-benzimidazol.

Beispiel 7

2-n-Propyl-4-methyl-6-(1-isopropyl-imidazol-4-yl)-1-[3-(2-hydroxycarbonyl-ethyl)-4-phenyl-benzyl]-benzimidazol

Hergestellt analog Beispiel 3 aus 2-n-Propyl-4-methyl-6-(1-isopropyl-imidazol-4-yl)-1-[3-(2,2-bis-hydroxycarbonyl-ethyl)-4-phenyl-benzyl]-benzimidazol.

Beispiel 8

2-Ethyl-5,7-dimethyl-3-[3-(tetrazol-5-yl-methyl)-4-phenyl-benzyl]-imidazo[4,5-b]pyridin

Hergestellt analog Beispiel 2 aus 2-Ethyl-5,7-dimethyl-3-(3-cyanomethyl-4-phenyl-benzyl]-imidazo[4,5-b]pyridin und Natriumazid in Dimethylformamid.
Ausbeute: 30 % der Theorie,
Schmelzpunkt: 209-211 °C

| $C_{25}H_{25}N_7$ (423,54) | | | | | | |
|---|---|---|---|---|---|---|
| Ber.:<br>Gef.: | C | 70,90<br>70,89 | H | 5,95<br>6,06 | N | 23,15<br>22,99 |

Massenspektrum: m/e = 423

Beispiel 9

2-n-Propyl-4-methyl-6-(1-isopropyl-imidazol-4-yl)-1-[3-(2-tetrazol-5-yl-ethyl)-4-phenyl-benzyl]-benzimidazol

Hergestellt analog Beispiel 4 aus 2-n-Propyl-4-methyl-6-(1-isopropyl-imidazol-4-yl)-1-[3-(2-cyano-ethyl)-4-phenyl-benzyl]-benzimidazol und Natriumazid in Dimethylformamid. Ausbeute: 50 % der Theorie,
Schmelzpunkt: ab 118 °C (sintern)
$C_{33}H_{36}N_8$ (544,71)
Massenspektrum: $(M+H)^+$ = 545
$R_f$-Wert: 0,33 (Kieselgel; Methylenchlorid/Ethanol = 9:1)
Bei den nachfolgenden pharmazeutischen Anwendungsbeispielen kann als Wirksubstanz jede geeignete Verbindung der Formel I, insbesondere diejenigen in denen $R_b$ eine in vivo in eine Carboxgruppe überführbare Gruppe, eine Carboxy- oder 1H-Tetrazolylgruppe darstellt, eingesetzt werden:

Beispiel I

Ampullen, enthaltend 50 mg Wirkstoff pro 5 ml

| | |
|---|---|
| Wirkstoff | 50 mg |
| $KH_2PO_4$ | 2 mg |
| $Na_2HPO_4$ x $2H_20$ | 50 mg |
| NaCl | 12 mg |
| Wasser für Injektionszwecke ad | 5 ml |

Herstellung:

In einem Teil des Wassers werden die Puffersubstanzen und das Isotonans gelöst. Der Wirkstoff wird zugegeben und nach vollständiger Lösung mit Wasser auf das Nennvolumen aufgefüllt.

Beispiel II

Ampullen, enthaltend 100 mg Wirkstoff pro 5 ml

| | |
|---|---|
| Wirkstoff | 100 mg |
| Methylglucamin | 35 mg |
| Glykofurol | 1000 mg |
| Polyethylenglykol-Polypropylenglykol-Blockpolymer | 250 mg |
| Wasser für Injektionszwecke ad | 5 ml |

Herstellung:

In einem Teil des Wassers wird Methylglucamin gelöst und der Wirkstoff unter Rühren und Erwärmen in Lösung gebracht. Nach Zugabe der Lösungsmittel wird mit Wasser auf das Nennvolumen aufgefüllt.

Beispiel III

Tabletten, enthaltend 50 mg Wirkstoff

| | |
|---|---|
| Wirkstoff | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Der Wirkstoff, $CaHPO_4$, Milchzucker und Maisstärke werden mit einer wässrigen PVP-Lösung gleichmäßig befeuchtet. Die Masse wird durch ein 2-mm-Sieb gegeben, im Umlufttrockenschrank bei 50 °C getrocknet und erneut gesiebt.

Nach Zumischen des Schmiermittels wird das Granulat auf einer Tablettiermaschine verpresst.

Beispiel IV

Dragees, enthaltend 50 mg Wirkstoff

| | |
|---|---|
| Wirkstoff | 50,0 mg |
| Lysin | 25,0 mg |
| Milchzucker | 60,0 mg |
| Maisstärke | 34,0 mg |
| Gelatine | 10,0 mg |
| Magnesiumstearat | 1,0 mg |
| | 180,0 mg |

Herstellung:

Der Wirkstoff wird mit den Hilfsstoffen gemischt und mit einer wässrigen Gelatine-Lösung befeuchtet. Nach Siebung und Trocknung wird das Granulat mit Magnesiumstearat vermischt und zu Kernen verpresst.

Die so hergestellten Kerne werden nach bekannten Verfahren mit einer Hülle überzogen. Der Dragiersuspension oder -lösung kann Farbstoff zugegeben werden.

Beispiel V

Dragees, enthaltend 100 mg Wirkstoff

| | |
|---|---|
| Wirkstoff | 100,0 mg |
| Lysin | 50,0 mg |
| Milchzucker | 86,0 mg |
| Maisstärke | 50,0 mg |
| Polyvinylpyrrolidon | 2,8 mg |
| Mikrokristalline Cellulose | 60,0 mg |
| Magnesiumstearat | 1,2 mg |
| | 350,0 mg |

Herstellung:

Der Wirkstoff wird mit den Hilfsstoffen gemischt und mit einer wässrigen PVP-Lösung befeuchtet. Die feuchte Masse wird durch ein 1,5-mm-Sieb gegeben und bei 45 °C getrocknet. Nach dem Trocknen wird erneut gesiebt und das Magnesiumstearat zugemischt. Diese Mischung wird zu Kernen verpreßt.

Die so hergestellten Kerne werden nach bekannten Verfahren mit einer Hülle überzogen. Der Dragiersuspension oder -lösung können Farbstoffe zugegeben werden.

Beispiel VI

Kapseln, enthaltend 250 mg Wirkstoff

| | |
|---|---|
| Wirkstoff | 250,0 mg |
| Maisstärke | 68,5 mg |
| Magnesiumstearat | 1,5 mg |
| | 320,0 mg |

Herstellung:

Wirkstoff und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel VII

Orale Suspension, enthaltend 50 mg Wirkstoff pro 5 ml

| | |
|---|---|
| Wirkstoff | 50,0 mg |
| Hydroxyethylcellulose | 50,0 mg |
| Sorbinsäure | 5,0 mg |
| Sorbit 70%ig | 600,0 mg |
| Glycerin | 200,0 mg |
| Aroma | 15,0 mg |
| Wasser ad | 5,0 ml |

Herstellung:

Destilliertes Wasser wird auf 70 °C erhitzt. Hierin wird unter Rühren Hydroxyethylcellulose gelöst. Durch Zugabe von Sorbitlösung und Glycerin wird auf Raumtemperatur abgekühlt. Bei Raumtemperatur werden Sorbinsäure, Aroma und Wirkstoff zugegeben. Zur Entlüftung der Suspension wird unter Rühren evakuiert. Eine Dosis = 50 mg ist enthalten in 5,0 ml.

Beispiel VIII

Suppositorien, enthaltend 100 mg Wirkstoff

| | |
|---|---|
| Wirkstoff | 100,0 mg |
| Adeps solidus | 1600,0 mg |
| | 1700,0 mg |

Herstellung:

Das Hartfett wird geschmolzen. Bei 40 °C wird die gemahlene Wirksubstanz in der Schmelze homogen dispergiert. Es wird auf 38 °C abgekühlt und in schwach vorgekühlte Suppositorienformen ausgegossen.

**Patentansprüche**

**1.** Substituierte Biphenylylderivate der allgemeinen Formel

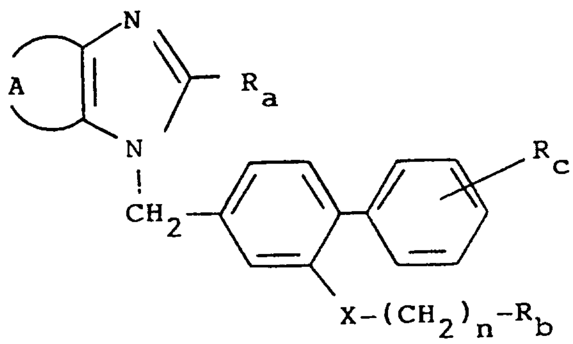

, (I)

in der
n die Zahl 0, 1, 2 oder 3 und
X eine Bindung oder
n die Zahl 1, 2 oder 3 und
X eine gegebenenfalls durch eine Alkylgruppe mit 1 bis 3 Kohlenstoffatomen substituierte Iminogruppe, ein Sauerstoff- oder Schwefelatom,
A eine 1,4-Butadienylengruppe, die durch die Reste $R_1$ und $R_2$ substituiert ist und in der zusätzlich eine unsubstituierte Methingruppe durch ein Stickstoffatom ersetzt sein kann, wobei
$R_1$ ein Wasserstoff-, Fluor-, Chlor- oder Bromatom, eine Trifluormethylgruppe oder eine Alkylgruppe mit 1 bis 3 Kohlenstoffatomen und
$R_2$ ein Wasserstoffatom,
eine Alkylgruppe mit 1 bis 3 Kohlenstoffatomen,
eine Alkoxygruppe mit 2 bis 5 Kohlenstoffatomen, die in 2-, 3-, 4- oder 5-Position durch eine Imidazolyl-, Benzimidazol-yl- oder Tetrahydrobenzimidazolylgruppe substituiert ist,
eine Alkanoylaminogruppe mit 2 bis 5 Kohlenstoffatomen im Alkanoylteil oder eine Benzolsulfonylaminogruppe, welche beide am Stickstoffatom durch eine Alkylgruppe mit 1 bis 3 Kohlenstoffatomen substituiert sein können,
eine Phthalimino- oder Homophthaliminogruppe, wobei eine Carbonylgruppe in einer Phthaliminogruppe durch eine Methylengruppe ersetzt sein kann,
eine 5-, 6- oder 7-gliedrige Alkyleniminogruppe, in welcher eine Methylengruppe durch eine Carbonyl- oder Sulfonylgruppe ersetzt sein kann,
eine Glutarsäureiminogruppe, in der die n-Propylengruppe durch ein oder zwei Alkylgruppen mit jeweils 1 bis 3 Kohlenstoffatomen oder durch eine Tetramethylen- oder Pentamethylengruppe substituiert sein kann,
eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,
eine gegebenenfalls in 1-Stellung durch eine Alkylgruppe mit 1 bis 6 Kohlenstoffatomen oder eine Cycloalkylgruppe mit 3 bis 7 Kohlenstoffatomen substituierte Benzimidazol-2-yl-gruppe, wobei der Phenylkern in einer vorstehend erwähnten Benzimidazol-2-ylgruppe zusätzlich durch ein Fluoratom, eine Methyl- oder Trifluormethylgruppe substituiert sein kann, eine Imidazo[2,1-b]thiazol-6-yl-, Imidazo-[1,2-a]pyridin-2-yl-, 5,6,7,8-Tetrahydro-imidazo[1,2-a]pyridin-2-yl-, Imidazo[1,2-a]pyrimidin-2-yl-, Imidazo[4,5-b]pyridin-2-yl-, Imidazo[4,5-c]pyridin-2-yl-, Imidazo[1,2-c]pyrimidin-2-yl-, Imidazo[1,2-a]-pyrazin-2-yl-, Imidazo[1,2-b)pyridazin-2-yl-, Imidazo[4,5-c]pyridin-2-yl-, Purin-8-yl-, Imidazo[4,5-b]-pyrazin-2-yl-, Imidazo[4,5-c]pyridazin-2-yl- oder Imidazo[4,5-d]pyridazin-2-yl-gruppe,
einen über ein Kohlenstoffatom gebundenen Pyrrolidin-, Piperidin- oder Pyridinring, wobei an den Pyridinring über zwei benachbarte Kohlenstoffatome ein Phenylrest ankondensiert und eine zum N-Atom benachbarte Methylengruppe in einem Pyrrolidin- oder Piperidinring durch eine Carbonyl- oder Sulfonylgruppe ersetzt sein kann,
eine gegebenenfalls in 2-Stellung durch eine Alkylgruppe mit 1 bis 6 Kohlenstoffatomen oder durch eine Phenylgruppe substituierte Imidazol-4-yl-Gruppe, die in 1-Stellung durch eine Alkylgruppe mit 1 bis 7 Kohlenstoffatomen, die in 2-, 3-, 4-, 5-, 6- oder 7-Stellung durch eine Carboxy-, Alkoxycarbonyl-, Aminocarbonyl-, Alkylaminocarbonyl-, Dialkylaminocarbonyl-, Morpholinocarbonyl-, Thiomorpholinocarbonyl- oder 1-Oxidothiomorpholinocarbonylgruppe substituiert sein kann, durch eine Alkylgruppe mit 1 bis 4 Kohlenstoffatomen, die in 2-, 3- oder 4-Stellung durch eine Hydroxy-, Alkoxy-

oder Imidazol-1-yl-Gruppe substituiert ist, durch eine Alkylgruppe, die durch eine Trifluormethylgruppe, durch eine Cycloalkylgruppe mit 3 bis 7 Kohlenstoffatomen oder durch eine gegebenenfalls durch Fluor- oder Chloratome, durch Trifluormethyl-, Methyl- oder Methoxygruppen mono- oder disubstituierte Phenylgruppe substituiert ist, durch eine durch zwei Phenylgruppen substituierte Alkylgruppe oder durch eine Cycloalkylgruppe mit 3 bis 7 Kohlenstoffatomen substituiert ist, wobei, soweit nichts anderes erwähnt wurde, die vorstehend erwähnten Alkyl- und Alkoxyteile jeweils 1 bis 3 Kohlenstoffatome enthalten können,

eine in vivo in eine Carboxygruppe überführbare Gruppe,

eine Carbonylgruppe, die durch eine Hydroxygruppe, durch eine Alkoxygruppe mit 1 bis 6 Kohlenstoffatomen, wobei die Methoxygruppe zusätzlich durch eine Alkanoyloxy-, Alkoxycarbonyloxy- oder Cycloalkoxycarbonyloxygruppe substituiert ist, oder durch eine gegebenenfalls durch Alkylgruppen mono- oder disubsituierte Aminogruppe substituiert ist, wobei in den vorstehend erwähnten Gruppen der Alkanoylteil 2 oder 3 Kohlenstoffatome, der Alkyl- und Alkoxyteil jeweils 1 bis 6 Kohlenstoffatome und der Cycloalkoxyteil 5 bis 7 Kohlenstoffatome enthalten kann, oder

eine $R_5$-$NR_4$-CO-$NR_3$-Gruppe, in der

$R_3$ ein Wasserstoffatom, eine Alkylgruppe mit 1 bis 5 Kohlenstoffatomen, eine Cyclohexyl- oder Benzylgruppe,

$R_4$ ein Wasserstoffatom, eine Alkylgruppe mit 1 bis 6 Kohlenstoffatomen, eine Allyl-, Cyclohexyl-, Benzyl- oder Phenylgruppe,

$R_5$ ein Wasserstoffatom oder eine Alkylgruppe mit 1 bis 3 Kohlenstoffatomen oder

$R_4$ und $R_5$ zusammen mit dem dazwischen liegenden Stickstoffatom eine unverzweigte Cycloalkyleniminogruppe mit 4 bis 6 Kohlenstoffatomen oder eine Morpholinogruppe oder

$R_3$ und $R_4$ zusammen eine Alkylengruppe mit 2 oder 3 Kohlenstoffatomen darstellen,

$R_a$ eine Alkylgruppe mit 1 bis 5 Kohlenstoffatomen, eine Cycloalkylgruppe mit 3 bis 5 Kohlenstoffatomen, eine Alkoxy-, Alkylthio- oder Alkylaminogruppe mit jeweils 1 bis 3 Kohlenstoffatomen in jedem Alkylteil,

$R_b$ eine in vivo in eine Carboxygruppe überführbare Gruppe, eine Carboxy-, Cyano-, Hydroxysulfonyl-, 1H-Tetrazolyl-, 1-Triphenylmethyl-tetrazolyl- oder 2-Triphenylmethyl-tetrazolylgruppe, eine Alkancarbonylaminosulfonyl-, Benzoylaminosulfonyl-, Alkansulfonylaminocarbonyl-, Trifluormethansulfonylaminocarbonyl-oder Phenylsulfonylaminocarbonylgruppe oder auch, wenn n die Zahl 1 und X eine Bindung darstellen, eine Bis(hydroxycarbonyl)methyl- oder Bis(alkoxcarbonyl)-methylgruppe, wobei in den vorstehend erwähnten Gruppen die Alkyl- und Alkoxyteile jeweils 1 bis 4 Kohlenstoffatome enthalten können, und

$R_c$ ein Wasserstoff-, Fluor-, Chlor- oder Bromatom, eine Alkylgruppe mit 1 bis 4 Kohlenstoffatomen, eine Alkoxy-, Nitro-, Amino-, Alkylamino- oder Dialkylaminogruppe mit jeweils 1 bis 3 Kohlenstoffatomen in jedem Alkylteil bedeuten,

wobei der Phenylkern der vorstehend erwähnten Phenylgruppen jeweils durch Chlor- oder Bromatome, Methyl- oder Methoxygruppen mono- oder disubstituiert sein kann und die Substituenten gleich oder verschieden sein können,

deren Gemische von Stellungsisomeren und deren Salze.

**2.** Substituierte Biphenylylderivate der allgemeinen Formel I gemäß Anspruch 1, in der

n die Zahl 0, 1, 2 oder 3 und

X eine Bindung oder

n die Zahl 1, 2 oder 3 und

X ein Sauerstoffatom,

A eine 1,4-Butadienylengruppe, die durch die Reste $R_1$ und $R_2$ substituiert ist und in der zusätzlich die Methingruppe in Position 7 des so gebildeten Benzimidazols durch ein Stickstoffatom ersetzt sein kann, wobei

$R_1$ ein Wasserstoffatom oder in 4-Stellung ein Fluor-, Chlor- oder Bromatom, eine Trifluormethylgruppe oder eine Alkylgruppe mit 1 bis 3 Kohlenstoffatomen und

$R_2$ ein Wasserstoffatom,

eine Alkylgruppe mit 1 bis 3 Kohlenstoffatomen,

in 6-Stellung eine Alkanoylaminogruppe mit 2 bis 5 Kohlenstoffatomen im Alkanoylteil oder eine Benzolsulfonylaminogruppe, welche beide am Stickstoffatom durch eine Alkylgruppe mit 1 bis 3 Kohlenstoffatomen substituiert sein können,

in 6-Stellung eine Phthalimino- oder Homophthaliminogruppe, wobei eine Carbonylgruppe in einer Phthaliminogruppe durch eine Methylengruppe ersetzt sein kann,

in 6-Stellung eine 5-, 6- oder 7-gliedrige Alkyleniminogruppe, in welcher eine Methylengruppe durch eine Carbonyl- oder Sulfonylgruppe ersetzt ist,
in 6-Stellung eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können,
in 6-Stellung eine gegebenenfalls in 1-Stellung durch eine Alkylgruppe mit 1 bis 6 Kohlenstoffatomen oder eine Cycloalkylgruppe mit 3 bis 7 Kohlenstoffatomen substituierte Benzimidazol-2-yl-gruppe, wobei der Phenylkern in einer vorstehend erwähnten Benzimidazol-2-ylgruppe zusätzlich durch ein Fluoratom, eine Methyl- oder Trifluormethylgruppe substituiert sein kann, eine Imidazo[2,1-b]thiazol-6-yl-, Imidazo[1,2-a]pyridin-2-yl-, 5,6,7,8-Tetrahydro-imidazo[1,2-a]pyridin-2-yl-, Imidazo[1,2-a]pyrimidin-2-yl- oder Imidazo[4,5-b]pyridin-2-yl-gruppe,
in 6-Stellung eine Imidazol-4-yl-Gruppe, die in 1-Stellung durch eine Alkylgruppe mit 1 bis 7 Kohlenstoffatomen, die in 2-, 3-, 4-, 5-, 6- oder 7-Stellung durch eine Carboxy-, Alkoxycarbonyl-, Aminocarbonyl-, Alkylaminocarbonyl-, Dialkylaminocarbonyl-, Morpholinocarbonyl-, Thiomorpholinocarbonyl- oder 1-Oxido-thiomorpholinocarbonylgruppe substituiert sein kann, durch eine Alkylgruppe mit 1 bis 4 Kohlenstoffatomen, die in 2-, 3- oder 4-Stellung durch eine Hydroxy-, Alkoxy- oder Imidazol-1-yl-Gruppe substituiert ist, durch eine Alkylgruppe, die durch eine Trifluormethylgruppe, durch eine Cycloalkylgruppe mit 3 bis 7 Kohlenstoffatomen oder durch eine gegebenenfalls durch Fluor- oder Chloratome, durch Trifluormethyl-, Methyl- oder Methoxygruppen mono- oder disubstituierte Phenylgruppe substituiert ist, durch eine durch zwei Phenylgruppen substituierte Alkylgruppe oder durch eine Cycloalkylgruppe mit 3 bis 7 Kohlenstoffatomen substituiert ist, wobei, soweit nichts anderes erwähnt wurde, die vorstehend erwähnten Alkyl- und Alkoxyteile jeweils 1 bis 3 Kohlenstoffatome enthalten können,
in 7-Stellung eine in vivo in eine Carboxygruppe überführbare Gruppe,
in 7-Stellung eine Carbonylgruppe, die durch eine Hydroxygruppe, durch eine Alkoxygruppe mit 1 bis 6 Kohlenstoffatomen, wobei die Methoxygruppe zusätzlich durch eine Alkanoyloxy-, Alkoxycarbonyloxy- oder Cycloalkoxycarbonyloxygruppe substituiert ist, oder durch eine gegebenenfalls durch Alkylgruppen mono- oder disubsituierte Aminogruppe substituiert ist, wobei in den vorstehend erwähnten Gruppen der Alkanoylteil 2 oder 3 Kohlenstoffatome, der Alkyl- und Alkoxyteil jeweils 1 bis 6 Kohlenstoffatome und der Cycloalkoxyteil 5 bis 7 Kohlenstoffatome enthalten kann, oder
in 6-Stellung eine $R_5$-$NR_4$-CO-$NR_3$-Gruppe, in der
$R_3$ ein Wasserstoffatom, eine Alkylgruppe mit 1 bis 5 Kohlenstoffatomen, eine Cyclohexyl- oder Benzylgruppe,
$R_4$ ein Wasserstoffatom, eine Alkylgruppe mit 1 bis 6 Kohlenstoffatomen, eine Allyl-, Cyclohexyl-, Benzyl- oder Phenylgruppe,
$R_5$ ein Wasserstoffatom oder eine Alkylgruppe mit 1 bis 3 Kohlenstoffatomen oder
$R_4$ und $R_5$ zusammen mit dem dazwischen liegenden Stickstoffatom eine unverzweigte Cycloalkyleniminogruppe mit 4 bis 6 Kohlenstoffatomen oder eine Morpholinogruppe oder
$R_3$ und $R_4$ zusammen eine Alkylengruppe mit 2 oder 3 Kohlenstoffatomen darstellen,
$R_a$ eine Alkylgruppe mit 2 bis 4 Kohlenstoffatomen, eine Cycloalkylgruppe mit 3 oder 4 Kohlenstoffatomen oder eine Alkoxygruppe mit 2 oder 3 Kohlenstoffatomen,
$R_b$ eine in vivo in eine Carboxygruppe überführbare Gruppe, eine Carboxy- oder 1H-Tetrazolylgruppe und
$R_c$ ein Wasserstoffatom bedeuten,
deren Gemische von Stellungsisomeren und deren Salze.

**3.** Substituierte Biphenylylderivate der allgemeinen Formel I gemäß Anspruch 1, in der
n die Zahl 0, 1, 2 oder 3 und
X eine Bindung,
A eine 1,4-Butadienylengruppe, die durch die Reste $R_1$ und $R_2$ substituiert ist und in der zusätzlich die Methingruppe in Position 7 des so gebildeten Benzimidazols durch ein Stickstoffatom ersetzt sein kann, wobei
$R_1$ ein Wasserstoffatom oder in 4-Stellung eine Methylgruppe und
$R_2$ in 6-Stellung eine 1-Isopropyl-imidazol-4-yl- oder 1-Methyl-benzimidazol-2-yl-Gruppe oder
in 7-Stellung eine in vivo in eine Carboxygruppe überführbare Gruppe oder eine Carboxygruppe darstellen,
$R_a$ eine n-Alkylgruppe mit 2 bis 4 Kohlenstoffatomen,
$R_b$ eine in vivo in eine Carboxygruppe überführbare Gruppe, eine Carboxy- oder 1H-Tetrazolylgruppe

und
$R_c$ ein Wasserstoffatom bedeuten,
deren Gemische von Stellungsisomeren und deren Salze.

**4.** Substituierte Biphenylylderivate der allgemeinen Formel I gemäß Anspruch 1, in der A, X, $R_a$ bis $R_c$ und n wie in den Ansprüchen 1 bis 3 mit der Maßgabe definiert sind, daß $R_b$ eine Carboxy- oder 1H-Tetrazol-5-yl-gruppe darstellt oder $R_2$ oder $R_b$ oder $R_2$ und $R_b$ eine in vivo in eine Carboxygruppe überführbare Gruppe der Formeln

- CO - OR',
- CO - O - (HCR'') - O - CO - R''' und
- CO - O - (HCR'') - O - CO - OR''' darstellen,

in denen
R' eine geradkettige oder verzweigte Alkylgruppe mit 1 bis 6 Kohlenstoffatomen, eine Cycloalkylgruppe mit 5 bis 7 Kohlenstoffatomen, eine Benzyl-, 1-Phenylethyl-, 2-Phenylethyl-, 3-Phenylpropyl-, Methoxymethyl- oder Cinnamylgruppe,
R'' ein Wasserstoffatom oder eine Methylgruppe und
R''' eine geradkettige oder verzweigte Alkylgruppe mit 1 bis 6 Kohlenstoffatomen, eine Cycloalkylgruppe mit 5 bis 7 Kohlenstoffatomen, eine Phenyl-, Benzyl-, 1-Phenylethyl-, 2-Phenylethyl- oder 3-Phenylpropylgruppe bedeuten,
deren Gemische von Stellungsisomeren und deren Salze.

**5.** Folgende Verbindungen der allgemeinen Formel I gemäß Anspruch 1:
(a) 2-n-Propyl-4-methyl-1-[3-(tetrazol-5-yl-methyl)-4-phenyl-benzyl]-benzimidazol,
(b) 2-n-Propyl-4-methyl-1-[3-(2-hydroxycarbonyl-ethyl)-4-phenyl-benzyl]-benzimidazol,
(c) 2-n-Propyl-4-methyl-6-(1-methyl-benzimidazol-2-yl)-1-[3-(2-hydroxycarbonyl-ethyl)-4-phenyl-benzyl]-benzimidazol,
(d) 2-Ethyl-5,7-dimethyl-3-[3-(tetrazol-5-yl-methyl)-4-phenyl-benzyl]-imidazo[4,5-b]pyridin und
(e) 2-n-Propyl-4-methyl-6-(1-isopropyl-imidazol-4-yl)-1-[3-(2-tetrazol-5-yl-ethyl)-4-phenyl-benzyl]-benzimidazol,
deren Gemische von Stellungsisomeren und deren Salze.

**6.** Physiologisch verträgliche Salze der Verbindungen nach mindestens einem der Ansprüche 1 bis 5 mit anorganischen oder organischen Säuren oder Basen.

**7.** Arzneimittel, enthaltend eine Verbindung nach mindestens einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

**8.** Verwendung einer Verbindung nach mindestens einem der Ansprüche 1 bis 6 zur Herstellung eines Arzneimittels mit Angiotensin-antagonistischer Wirkung.

**9.** Verfahren zur Herstellung eines Arzneimittels gemäß Anspruch 7, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

**10.** Verfahren zur Herstellung der substituierten Biphenylylderivate der allgemeinen Formel gemäß den Ansprüchen 1 bis 6, dadurch gekennzeichnet, daß
a) ein Benzimidazol der allgemeinen Formel

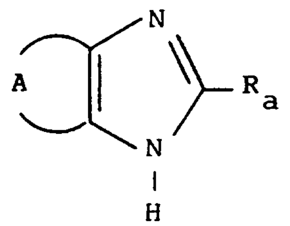

,(II)

in der
A und $R_a$ wie in den Ansprüchen 1 bis 5 definiert sind, mit einer Biphenylverbindung der allgemeinen Formel

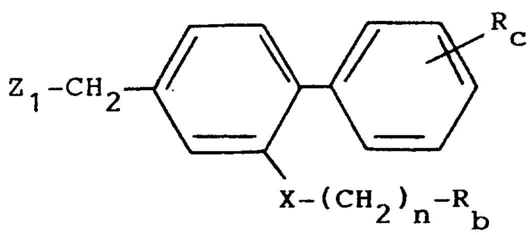

,(III)

in der
n, X, $R_b$ und $R_c$ wie in den Ansprüchen 1 bis 5 definiert sind und
$Z_1$ eine nukleophile Austrittsgruppe darstellt, umgesetzt und gegebenenfalls eine so erhaltene Verbindung hydrolysiert wird oder
b) zur Herstellung einer Verbindung der allgemeinen Formel I, in der $R_b$ eine Carboxygruppe darstellt, eine Verbindung der allgemeinen Formel

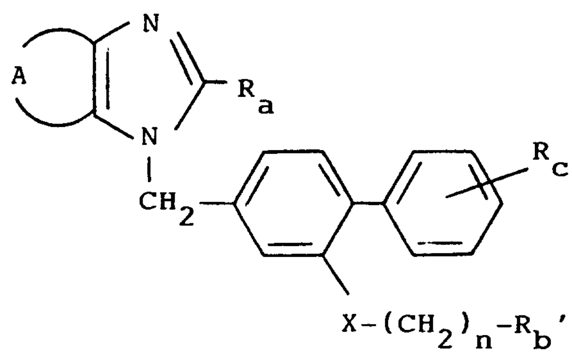

,(IV)

in der
n, X, A, $R_a$ und $R_c$ wie in den Ansprüchen 1 bis 5 definiert sind und
$R_b'$ eine mittels Hydrolyse, Thermolyse oder Hydrogenolyse in eine Carboxygruppe überführbare Gruppe darstellt, in eine entsprechende Carboxyverbindung übergeführt wird oder
c) zur Herstellung einer Verbindung der allgemeinen Formel I, in der $R_b$ eine 1H-Tetrazolylgruppe darstellt, ein Schutzrest von einer Verbindung der allgemeinen Formel

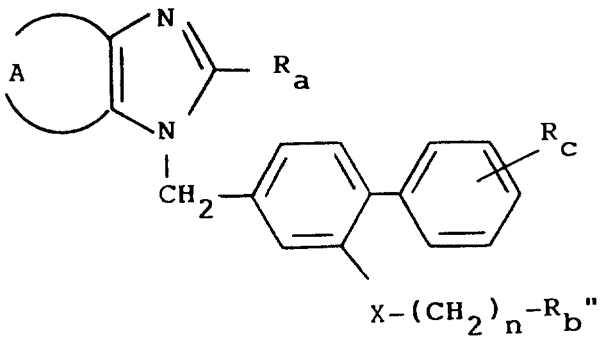

,(V)

in der

n, X, A, $R_a$ und $R_c$ wie in den Ansprüchen 1 bis 5 definiert sind und

$R_b$" eine in 1- oder 2-Stellung durch einen Schutzrest geschützte 1H-Tetrazolylgruppe darstellt, abgespalten wird oder

d) zur Herstellung einer Verbindung der allgemeinen Formel I, in der $R_b$ eine 1H-Tetrazolylgruppe darstellt, eine Verbindung der allgemeinen Formel

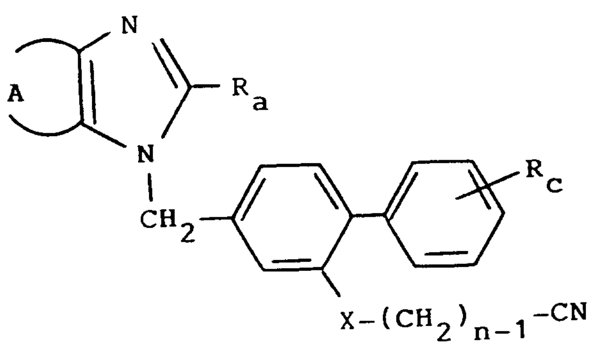

,(VI)

in der

n, X, A, $R_a$ und $R_c$ wie in den Ansprüchen 1 bis 5 definiert sind, mit Stickstoffwasserstoffsäure oder mit deren Salzen umgesetzt wird oder

e) zur Herstellung einer Verbindung der allgemeinen Formel I, in der n die Zahl 1, X eine Bindung und $R_b$ eine Bis(hydroxycarbonyl)methyl- oder Bis(alkoxycarbonyl)methyl-Gruppe darstellen, eine Verbindung der allgemeinen Formel

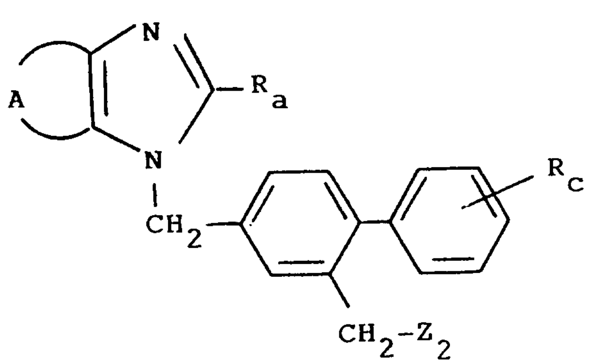

,(VII)

in der

n, A, $R_a$ und $R_c$ wie in den Ansprüchen 1 bis 5 definiert sind und

$R_2$ eine nukleophile Austrittsgruppe bedeuten, mit einer Verbindung der allgemeinen Formel

$CH_2(COOR_6)_2$ ,(VIII)

in der

$R_6$ jeweils eine Alkylgruppe mit 1 bis 5 Kohlenstoffatomen darstellt, umgesetzt und erforderlichenfalls anschließend eine so erhaltene Verbindung hydrolysiert und/oder decarboxyliert wird oder

f) zur Herstellung einer Verbindung der allgemeinen Formel I, in der $R_2$ eine $R_5$-$NR_4$-$CONR_3$-Gruppe darstellt, eine Verbindung der allgemeinen Formel

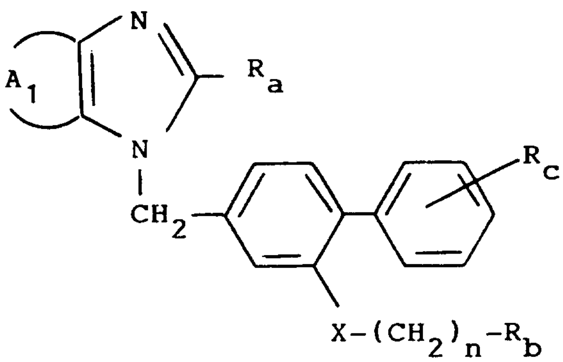

,(IX)

mit einer Verbindung der allgemeinen Formel

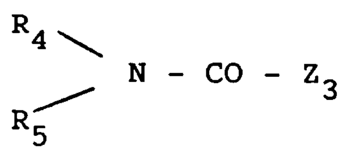

,(X)

in denen

$R_a$, $R_b$, $R_c$, $R_4$, $R_5$, X und n wie in den Ansprüchen 1 bis 5 definiert sind,

$A_1$ eine 1,4-Butadienylengruppe, die durch $R_1$ und durch die $R_3$NH-Gruppe substituiert ist, wobei $R_1$ und $R_3$ wie in den Ansprüchen 1 bis 5 definiert sind, und

$Z_3$ eine nukleophile Austrittsgruppe oder auch $Z_3$ und $R_5$ zusammen eine Stickstoff-Kohlenstoff-Bindung darstellen, umgesetzt wird oder

g) zur Herstellung einer Verbindung der allgemeinen Formel I, in der $R_2$ eine Alkanoylaminogruppe mit 2 bis 5 Kohlenstoffatomen im Alkanoylteil oder eine Benzolsulfonylaminogruppe, welche beide am Stickstoffatom durch eine Alkylgruppe mit 1 bis 3 Kohlenstoffatomen substituiert sein können, eine Phthalimino- oder Homophthaliminogruppe, wobei eine Carbonylgruppe in einer Phthaliminogruppe durch eine Methylengruppe ersetzt sein kann, eine 5-, 6- oder 7-gliedrige Alkyleniminogruppe, in welcher eine Methylengruppe durch eine Carbonyl- oder Sulfonylgruppe ersetzt ist, eine Glutarsäureiminogruppe, in der die n-Propylengruppe durch ein oder zwei Alkylgruppen mit jeweils 1 bis 3 Kohlenstoffatomen oder durch eine Tetramethylen- oder Pentamethylengruppe substituiert sein kann, oder eine gegebenenfalls durch eine Alkylgruppe mit 1 bis 3 Kohlenstoffatomen oder durch eine Phenylgruppe mono- oder disubstituierte Maleinsäureimidogruppe, wobei die Substituenten gleich oder verschieden sein können, darstellt, eine Verbindung der allgemeinen Formel

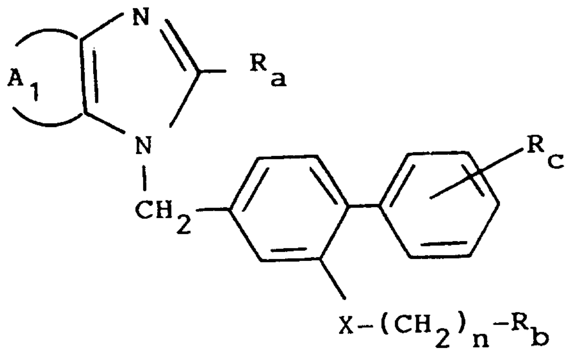

,(IX)

mit einer Verbindung der allgemeinen Formel

$Z_4$ - U - $R_7$ ,(XI)

in denen

$R_a$, $R_b$, $R_c$, $R_4$, $R_5$, X und n wie in den Ansprüchen 1 bis 5 definiert sind,

$A_1$ eine 1,4-Butadienylengruppe, die durch $R_1$ und durch die $R_3$NH-Gruppe substituiert ist, wobei $R_1$ und $R_3$ wie in den Ansprüchen 1 bis 5 definiert sind,

$Z_4$ eine Hydroxygruppe oder eine nukleophile Austrittsgruppe,

U eine Carbonyl- oder Sulfonylgruppe und

$R_7$ eine Alkylgruppe mit 1 bis 5 Kohlenstoffatomen, eine Phenyl-, o-Hydroxycarbonylphenyl-, o-Hydroxycarbonylphenylmethyl- oder o-Hydroxycarbonylmethylphenylgruppe, eine gegebenenfalls durch eine oder zwei Alkylgruppen mit jeweils 1 bis 3 Kohlenstoffatomen oder durch eine Tetramethylen- oder Pentamethylengruppe substituierte 3-Hydroxycarbonylpropylengruppe oder eine gegebenenfalls durch eine Alkylgruppe mit 1 bis 3 Kohlenstoffatomen oder durch eine Phenylgruppe mono- oder disbustituierte 2-Hydroxycarbonylethenylengruppe, in der die Substituenten gleich oder verschieden sein können, oder $R_3$ und $R_7$ zusammen eine n-Propylen-, n-Butylen- oder n-Hexylengruppe bedeuten, oder, falls $Z_4$ eine Hydroxygruppe darstellt, mit deren reaktionsfähigen Derivaten umgesetzt wird und

gewünschtenfalls anschließend eine so erhaltene Verbindung der allgemeinen Formel I, in der $R_2$ oder $R_b$ oder $R_2$ und $R_b$ jeweils eine Carboxygruppe darstellen, mittels Veresterung in eine entsprechende Verbindung der allgemeinen Formel I, in der $R_2$ oder $R_b$ oder $R_2$ und $R_b$ eine in vivo in eine Carboxygruppe überführbare Gruppe darstellen, übergeführt wird und

erforderlichenfalls ein während der Umsetzungen a) bis g) zum Schutze von reaktiven Gruppen verwendeter Schutzrest abgespalten wird und/oder

gewünschtenfalls anschließend ein so erhaltenes Gemisch von Stellungsisomeren einer Verbindung der allgemeinen Formel I mittels Isomerentrennung aufgetrennt wird oder

eine so erhaltene Verbindung der allgemeinen Formel I in ihr Salz, insbesondere für die pharmazeutische Anwendung in ihr physiologisch verträgliches Salz mit einer anorganischen oder organischen Säure oder Base, übergeführt wird.